# FASCICULE DE BREVET EUROPEEN

(11) **EP 0 835 309 B1**
(45) Date de publication et mention de la délivrance du brevet: **04.10.2001**
(21) Numéro de dépôt: 96924033.2
(22) Date de dépôt: 28.06.1996
(51) Int. Cl.: C12N 15/12, A61K 39/21, A61K 39/385, A61K 39/395, C07K 16/28, G01N 33/577

(54) **VACCIN CONTRE DES AGENTS INFECTIEUX, COMPOSITION POUR LE TRAITEMENT ET LA PREVENTION DES INFECTIONS A HIV**
IMPSTOFF GEGEN KRANKHEITSERREGER, ZUSAMMENSETZUNG ZUR BEHANDLUNG UND VORBEUGUNG VON HIV INFEKTIONEN
VACCINE FOR INFECTIOUS AGENTS, COMPOSITION FOR TREATING AND PREVENTING HIV INFECTIONS

(30) Priorité: 30.06.1995 FR 9507914
(43) Date de publication de la demande: 15.04.1998
(60) Demande divisionnaire: 01106012.6
(73) Titulaire: INSTITUT NATIONAL DE LA SANTE ET DE LA RECHERCHE MEDICALE (INSERM), 75013 Paris (FR)
(72) Inventeur: CHERMANN, Jean-Claude, F-13260 Cassis (FR); LE CONTEL, Carole, F-13001 Marseille (FR); GALEA, Pascale, F-13009 Marseille (FR)
(74) Mandataire: Warcoin, Jacques
(86) Numéro de dépôt international: FR9601006
(87) Numéro de publication internationale: WO9702344

(56) Documents cités:
- WO-A-92/18630
- WO-A-93/14126
- WO-A-94/01130
- WO-A-94/24290
- CELLULAR PHARMACOLOGY 3 (2). 1996. 68-73, XP000616506 LE CONTEL C ET AL: "Identification of the beta-2m derived epitope responsible for neutralization of HIV isolates."
- The Journal of Immunology, 127(4), 1981, 1542-8

## Description

La présente invention concerne de nouveaux types de vaccins et notamment des compositions destinées au traitement, à la prévention et au diagnostic des affections à HIV.

Plus spécifiquement, la présente invention concerne des peptides capables de produire une réponse immunitaire susceptible de directement ou indirectement neutraliser les virus HIV chez les mammifères et notamment chez l'homme.

On a déjà décrit, notamment dans le brevet EP-B- 0470989 ainsi que dans différentes publications, l'importance des anticorps monoclonaux dirigés contre la β2 microglobuline (β2m) dans l'inhibition de la réplication de HIV-1.

En particulier, il a pu être mis en évidence que ces anticorps agissaient sur deux mécanismes, à savoir, directement sur le virus et sur les cellules associées à la β2m.

La présente invention constitue des développements de ces éléments préliminaires et repose sur la mise en évidence de séquences peptidiques provenant de β2m ou ayant une structure équivalente susceptibles de générer des anticorps neutralisant totalement ou partiellement les virus HIV.

Compte tenu de la complexité des mécanismes mis en oeuvre on entendra par "neutralisation du virus HIV" tout mécanisme ayant pour effet in vivo de détruire et/ou d'empêcher la propagation des virus.

In vitro, ces anticorps neutralisants peuvent être utilisés pour neutraliser tout fluide corporel destiné à être réinoculé ou réintroduit chez l'homme, comme le sperme d'un homme HIV séropositif pour l'insémination d'une femme séronégative.

Mais, plus généralement, la présente invention repose sur une nouvelle approche vaccinale utilisable, en particulier, pour les agents infectieux type parasite ou virus à fort pouvoir de mutation. En effet, dans le cadre de la vaccination traditionnelle on cherche à générer des anticorps neutralisants dirigés contre des éléments de l'agent infectieux, mais lorsque celui-ci présente un fort pouvoir de mutation, tel que le HIV par exemple, cette stratégie ne donne, au mieux, que des résultats limités pour un isolat particulier qui sera remplacé très vite par un isolat mutant et résistant.

La nouvelle approche vaccinante repose sur un concept différent et est applicable à un certain nombre d'agents infectieux qui présentent une phase intracellulaire lors de leur cycle.

En effet, on sait pour certains agents, ou on peut mettre en évidence, notamment dans le cas de HIV, ce qui constitue une partie de la présente invention, que, lors de la multiplication des agents infectieux à partir des cellules infectées de l'hôte, les agents infectieux extracellulaires emportent des éléments de déterminants de la cellule hôte.

L'un des objets de la présente invention consiste à prendre comme cible, non pas l'agent infectieux lui-même, mais les éléments du déterminant qu'il emporte avec lui et de prévoir une vaccination dirigée contre ces déterminants cellulaires qui resteront constants, même si l'agent lui-même a muté.

Ce type d'approche présente bien entendu une limite immédiate, l'antigène étant lié aux cellules hôtes, il n'est possible de réaliser une telle vaccination qu'avec un épitope cryptique du déterminant cellulaire qui ne sera dévoilé que lorsqu'il sera emporté par l'agent infectieux extracellulaire, ou un épitope non-immunogène dans sa présentation naturelle par la cellule et modifié lorsqu'il est présenté à la surface du virion.

Dans le cas de HIV par exemple, on a pu mettre en évidence que la β2 microglobuline présentait plusieurs épitopes cryptiques, lesquels étaient dévoilés lors de la multiplication du virus HIV et son passage à l'extérieur de la cellule. Il n'y a donc pas, en cas de vaccination, d'une part de réaction auto-immune, et, d'autre part, l'épitope étant lié aux différents isolats de HIV qui ont été testés, la vaccination est efficace, et ceci indépendamment des mutations du virus lui-même.

Ce type de vaccination peut être retenu, notamment, pour les parasites intracellulaires et les virus enveloppés tels que CMV, HPV, HSV et HIV par exemple.

Il faut bien comprendre que si ce type de vaccination n'est pas utilisable dans tous les cas, il peut constituer une alternative très intéressante pour les agents infectieux résistant aux approches plus traditionnelles.

C'est pourquoi la présente invention concerne un vaccin contre un agent infectieux, caractérisé en ce qu'il comporte au moins un épitope cryptique d'un élément cellulaire emporté par un agent infectieux intracellulaire lors de son passage à l'extérieur de la cellule et qui est dévoilé par l'agent infectieux.

De préférence, cet agent infectieux est un parasite ou un virus à enveloppe et l'épitope cryptique est situé au voisinage de la surface de la cellule.

Par "épitope cryptique", on entend désigner un épitope d'un déterminant cellulaire de l'hôte qui est caché ou modifié et est donc reconnu comme étranger par le système immunitaire et ne produit donc pas de réaction auto-immune avec destruction du déterminant correspondant et peut être utilisé pour la vaccination.

L'épitope cryptique doit évidemment être dévoilé, c'est-à-dire être accessible et reconnu par le système immunitaire lorsqu'il est emporté par l'agent infectieux (dans le cas où il resterait cryptique, la vaccination ne serait pas possible).

Dans le cas de la β2 microglobuline, on a pu mettre en évidence l'existence de ce type d'épitope qui d'ailleurs se découvre également sous forme naturelle lors de l'élimination de la β2 microglobuline par voie urinaire.

La présente invention concerne donc des compositions destinées au traitement ou à la prévention des infections à HIV, caractérisées en ce qu'elles comportent à titre de principe actif au moins un peptide correspondant aux séquences 1 à 22 ou une séquence équivalente. Par "séquence équivalente", on entend désigner une séquence qui lève la neutralisation du virus HIV par les anticorps monoclonaux B1G6 ou B262.2 in vitro.

B1G6 et B262.2 sont décrits dans Liabeuf et al (1981); The Journal of Immunology, Vol 127, N°4 1542:1548.

Ces peptides constituent des épitopes cryptiques de la β2 microglobuline comme décrits précédemment.

Les peptides selon la présente invention sont les suivants :
01 - P1 IQRTPKIQVYSRHRA
   (Ile-Gln-Arg-Thr-Pro-Lys-Ile-Gln-Val-Tyr-Ser-Arg-His-Pro-Ala)
02 - P4 FHPSDIEVDLLKDGE
   (Phe-His-Pro-Ser-Asp-Ile-Glu-Val-Asp-Leu-Leu-Lys-Asp-Gly-Glu)
03 - P9 ACRVNHVTLSQPKIV
   (Ala-Cys-Arg-Val-Asn-His-Val-Thr-Leu-Ser-Gln-Pro-Lys-Ile-Val)

On peut également utiliser une partie plus petite (7 amino-acides) de ces 15 amino-acides qui lève la neutralisation du virus par les anticorps monoclonaux B1G6 ou B2G2.2 :
04 - R-7-V RTPKIQV (Arg-Thr-Pro-Lys-Ile-Gln-Val)
05 - S-7-K SQPKIVK (Ser-Gln-Pro-Lys-Ile-Val-Lys)
06 - F-7-E FHPSDIE (Phe-His-Pro-Ser-Asp-Ile-Glu)

Une structure commune PKI (3 amino acides) semble être le motif responsable ; d'où les modifications d'amino-acides suivantes :
07 - TLSRTPKIQV (Thr-Leu-Ser-Arg-Thr-Pro-Lys-Ile-Gln-Val) n° 185
08 - IYLTQPKIKV (Ile-Tyr-Leu-Thr-Gln-Pro-Lys-Ile-Lys-Val) n° 186
09 - IQRTPKIQVY (Ile-Gln-Arg-Thr-Pro-Lys-Ile-Gln-Val-Tyr) n° 187
10 - TLSQPKIVKN (Thr-Leu-Ser-Gln-Pro-Lys-Ile-Val-Lys-Asn) n° 188
11 - IQRTPQIVKW (Ile-Gln-Arg-Thr-Pro-Gln-Ile-Val-Lys-Trp) n° 189
12 - IQRTPNIVKW (Ile-Gln-Arg-Thr-Pro-Asn-Ile-Val-Lys-Trp) n° 190

On peut également introduire une cystéine et un site de glycosylation :
13 - CYNPSDIE (Cys-Tyr-Asn-Pro-Ser-Asp-Ile-Glu)
14 - YCNPEST (Tyr-Cys-Asn-Pro-Glu-Ser-Thr)
15 - NFLNCYVS (Asn-Phe-Leu-Asn-Cys-Tyr-Val-Ser)
16 - LNCYVSPSD (Leu-Asn-Cys-Tyr-Val-Ser-Pro-Ser-Asp)

Enfin, on peut utiliser les peptides utilisant les différentes variations en fonction des espèces (souris, primates, lapins, cobayes) :
17 - KTPQJQV (Lys-Thr-Pro-Gln-Ile-Gln-Val)
18 - FHPPQJE (Phe-His-Pro-Pro-Gln-lle-Glu)
19 - FHPPHIE (Phe-His-Pro-Pro-His-Ile-Glu)
20 - AEPKTVY (Ala-Glu-Pro-Lys-Thr-Val-Tyr)
21 - SQPKTVY (Ser-Gln-Pro-Lys-Thr-Val-Tyr)
22 - ILSRTPKIQV (Ile-Leu-Ser-Arg-Thr-Pro-Lys-Ile-Gln-Val)

Ces peptides de SEQ ID 1 à 22 ne contiennent que le choix préférentiel, il est possible comme cela a été indiqué précédemment de trouver des peptides équivalents.

L'exemple 5 décrit un procédé permettant de mettre en évidence les peptides équivalents.

Ces peptides sont, de préférence, liés à un système porteur ; il peut s'agir soit d'un ou plusieurs fragments de protéine liés à l'extrémité N et/ou C terminale desdits peptides afin de permettre notamment une réponse immunitaire, on parlera alors de "protéines conjuguées". Parmi les protéines utilisables il faut citer surtout les albumines, la KLH ("Keyhole Limpet Hemocyanin") la MAP ("Multiple Antigenic Peptide") ou d'autres protéines connues pour leur pouvoir immunogène. Il est possible également de prévoir des protéines ou des fragments de protéine liés par des liaisons non peptidiques telles que pont disulfure ou liaisons par ion calcium.

Lors de l'étude des différents peptides selon l'invention, il est apparu, bien qu'il ne s'agisse là que d'une théorie qui ne saurait en aucune façon limiter la présente invention, que la structure PKI joue un rôle essentiel. En effet, la proline est un amino-acide qui impose une conformation et qui limite la possibilité d'une configuration peptidique quaternaire. Dans ces conditions KI (Lys, Ile) est fixé dans une position qui est exposée à réagir avec un anticorps.

Dans ces conditions, lors de la construction des protéines support, il conviendra de prévoir de préférence une structure qui laisse accessible la structure PKI.

L'analyse de la structure des régions sélectionnées pour P1, P9 et P10 peut être effectuée par des méthodes telles que la sélection utilisant l'alanine pour remplacer chaque amino-acide séparément, notamment dans la région RTPKIQV, afin de mettre en évidence les acides aminés éventuels. On peut également utiliser les techniques mettant en oeuvre la biotinilation de chaque peptide puis la sélction par EIA avec les anticorps pour mettre en évidence la perte de fixation.

Il est ainsi possible de prévoir de conjuguer les épitopes en cause avec des composants non protéiques, par exemple des polysaccharides et/ou des lipides, pour constituer des lipoprotéines présentant des activités vaccinantes améliorées ; là encore, il est possible de prévoir des liaisons covalentes ou non.

Ces différents types de composés peuvent être obtenus, soit par synthèse chimique, soit par des voies recombinantes mettant en oeuvre des techniques connues dans le domaine de la production des protéines recombinantes.

La souplesse des technologies recombinantes permet de réaliser des protéines comportant une pluralité d'épitopes identiques ou différents et susceptibles d'améliorer l'activité du produit final. Il est également possible de prévoir la co-expression de différents éléments entrant dans les compositions selon l'invention.

Selon l'un des aspects de l'invention, le peptide pourra être introduit dans une protéine de structure connue du HIV ; en particulier des constructions dans lesquelles le peptide d'intérêt est inséré dans la région hypervariable de la boucle V3 de la gp120 peuvent être utilisées.

La région V3 de la gp120 est le principal domaine de neutralisation de HIV-1 et l'un des déterminants majeurs du tropisme viral. En conséquence, ce type de mutant peut être utile pour étudier la neutralisation de HIV-1 liée au R7V et les modifications de son spectre d'hote. La haute variabilité de la région V3 de la gp120 parmi les isolats de HIV-1 est une autre raison de la préférence pour cette région. Il a été supposé que le remplacement de la séquence de sept amino-acides dans la région V3 avait de plus fortes chances de conduire à un recombinant viable qu'une mutation dans une autre région plus conservatrice du génôme de HIV-1. La protéine recombinante gp120/R7V peut être exprimée en parallèle dans un système convenable d'expression d'une protéine pour obtenir une grande quantité d'immunogène R7V.

L'utilisation de protéines porteuses n'est pas indispensable, il est possible de prévoir éventuellement d'autres systèmes porteurs. Par "système porteur", on entend désigner tout élément qui permet de conduire à un ensemble générant une réponse immunitaire contre le peptide en cause, ou bien qui permet de protéger le peptide d'une élimination, notamment d'une protéolyse rapide.

Les compositions selon l'invention peuvent comporter également des composants augmentant l'immunogénicité des peptides et/ou protéines, notamment des adjuvants d'immunité spécifiques ou non tels que l'adjuvant de Freund, des polysaccharides ou des composés équivalents.

Il s'agit là de procédés qui sont connus dans le domaine de la vaccination.

Les compositions selon l'invention peuvent être utilisées sous une forme quelconque compatible avec la voie d'administration choisie, en particulier la voie injectable. Toutefois les compositions selon la présente invention pourront être utilisées par d'autres voies, notamment per os ou par voie aérosol, pour induire une protection des muqueuses.

La présente invention concerne également des compositions destinées à être administrées afin d'exprimer in situ les peptides et/ou protéines décrites précédemment.

En particulier, la présente invention concerne des cassettes d'expression d'ADN permettant d'exprimer au moins un épitope cryptique tel que défini précédemment, et/ou les protéines présentant ces épitopes ou des protéines susceptibles de se coupler avec le peptide en cause tel que défini précédemment ou ayant des séquences équivalentes.

Par "séquence équivalente", on entend désigner une séquence codant pour un peptide équivalent tel que cela a été décrit précédemment.

Ces cassettes d'expression d'ADN peuvent, bien entendu, être utilisées soit directement pour une expression in situ, soit être utilisées pour produire un peptide ou une protéine utilisable comme cela a été décrit précédemment.

Les systèmes de vaccination mettant en oeuvre des séquences d'ADN sont connus et sont déjà largement décrits dans la littérature.

Il s'agit essentiellement de systèmes permettant l'expression de la protéine antigénique chez l'homme, ou bien l'expression de la protéine antigénique dans une cellule, laquelle est ensuite utilisée pour la vaccination ; lorsque la cellule transformée est une cellule de l'hôte traitée à l'extérieur on partle de traitement ex vivo.

Les systèmes d'expression peuvent être très variés, il pourra s'agir notamment de systèmes de type "ADN nu " tels qu'ils sont décrits notamment dans les brevets et demandes de brevet de la société VICAL, WO 90/11092. Dans ce cas, l'ADN codant pour le peptide ou la protéine comportant le peptide est injecté tel quel, cette injection conduit dans un certain nombre de cas à l'expression de la protéine codée.

Les informations contenues dans ces documents sont incluses explicitement dans la présente description par référence.

On pourra utiliser également des systèmes d' "ADN nu" mais comportant leur propre système d'expression notamment afin d'améliorer l'expression.

On pourra également utiliser des systèmes favorisant l'expression, soit par intégration, soit par réplication autonome, notamment des systèmes de type plasmidique ou viral.

Parmi les systèmes d'expression de séquence peptidique que l'on peut mentionner, il faut citer les systèmes utilisant des poxvirus, des adénovirus, des rétrovirus et des virus de type herpès ou d'autres systèmes plus récents tels que les poliovirus.

Parmi les vecteurs, on utilisera de préférence les vecteurs générant une réponse humorale et pour les muqueuses.

D'autres virus peuvent être utilisés afin d'obtenir des vaccins en particulier :
- les adénovirus comme cela est décrit dans N.R. Rabinovich et al. Science, 1994, 265, 1401-1404 et références citées ;
- les rotavirus comme cela est décrit par Sue E Crowford, dans Journal of Virology, Sept. 1994, p. 5945-5952;
- les poxvirus, notamment la vaccine virus, également les poxvirus animaux comme le canari pox tel que cela est décrit dans les travaux de Paoletti et de Moss ;
- influenza virus tel que décrit dans N.R. Rabinovich et al. (1994).

La technologie permettant d'utiliser les poliovirus à titre de vecteur de vaccination pour différents antigènes est décrite notamment dans Raul Andino et al, Science, 265, 1448-51.

Ce type de construction utilisable dans le cadre de la présente invention permet d'obtenir des vaccins utilisables par voie orale ; pour ce faire on clone la séquence codant pour le ou les peptides éventuellement les protéines porteuses dans un poliovirus, par exemple le virus Sabin atténué, il est possible également d'utiliser un cocktail de virus codant pour différents épitopes.

L'utilisation de plasmides ou de virus pour l'expression de protéines dans les cellules d'un hôte, notamment humain, est connue et ne sera pas explicitée en détail. Les constructions spécifiques dépendent évidemment de l'hôte, de l'épitope et du vecteur retenu.

On peut également utiliser des vaccins cellulaires, c'est-à-dire, par exemple comme cela est proposé dans le cadre de la thérapie génique, de prélever des cellules du patient, les transformer avec des vecteurs tels que décrits précédemment puis les réimplanter afin d'exprimer les protéines in situ.

Toutefois, dans le cas d'une vaccination, ce procédé est peu commode. On préférera utiliser des cellules qui peuvent être obtenues en grand nombre, cellules bactériennes ou de levure par exemple, qui expriment la protéine en cause, par exemple en surface, ce qui dans certains cas augmente le pouvoir immunogène de la protéine.

Il est possible, par exemple, d'utiliser les vaccins comportant comme système d'expression Salmonella comme cela est décrit dans T.R. Fouts et al., Vaccine (1995) 13 in press ; Tacket C.O. et al., Infect. Immun. (1992) 60, 536-541 et Hone et al., J. Chim. Invest. (1992) 90, 412-420 (pour son évaluation chez l'homme comme support vaccinal).

Ce type de vaccin implique l'utilisation de cellules notamment bactériennes produisant les peptides selon l'invention ou certaines souches d'autres vecteurs de vaccination et décrit dans Chad P Muller, Immunology Today. vol. 15 n° 20. 1994, p. 458-459.

Les cellules produisant les peptides ou protéines selon l'invention peuvent être utilisées telles quelles en particulier lorsque les protéines sont exprimées à la surface des cellules et lorsque les cellules sont non toxiques et non pathogènes (souche atténuée ou tuée), mais peut également être utilisé pour produire les peptides et/ou protéines qui seront utilisés après purification.

Ainsi, il peut être intéressant d'obtenir des cellules bactériennes mais également des levures ou des cellules supérieures, cellules animale, végétale ou d'insecte notamment.

Dans le cas de la présente invention, on peut prévoir l'utilisation de vaccins d'origine végétale en utilisant les technologies décrites notamment dans C.J. Arntzel et al. dans Vaccine 94.

Les technologies permettant l'expression des peptides ou protéines par des systèmes cellulaires sont connues de même que les techniques de purification.

Comme cela a déjà été mentionné, il est possible d'utiliser les compositions selon l'invention avec des adjuvants améliorant l'activité des séquences d'ADN, notamment des composants constituant des complexes avec l'ADN, tels que les lipides cationiques, ou des structures de type liposome ou microparticule.

L'invention concerne également des compositions contenant des anticorps contre les peptides selon l'invention.

Bien entendu l'utilisation de compositions à base d'anticorps nécessite que ceux-ci soient compatibles avec l'administration à l'être humain ; il peut notamment s'agir d'anticorps humanisés par des techniques connues ou directement exprimés in situ à partir de la séquence d'ADN.

La présente invention concerne également l'utilisation des anticorps dressés contre les peptides de l'invention et susceptibles de neutraliser le virus HIV, en particulier la présente invention concerne des anti-sérums comportant ce type d'anticorps ou les anticorps obtenus, par exemple par immunopurification, à partir desdits sérums.

La présente invention concerne également un procédé de diagnostic, caractérisé en ce qu'on met en évidence dans le sérum d'un patient la présence d'un anticorps dirigé contre l'un des épitopes selon l'invention.

Ce procédé peut être mis en oeuvre par toute méthode connue d'identification des anticorps, notamment les méthodes ELISA et RIA et toutes les méthodes qui en dérivent.

Toutes ces méthodes reposent de préférence sur la fixation des anticorps en cause sur les peptides antigéniques décrits précédemment puis mise en évidence de cette fixation. Ce diagnostic présente un intérêt considérable, en effet les exemples montrent que les séropositifs dans le cas de HIV qui présentent des anticorps selon l'invention sont dans un très grand nombre de cas non progresseurs, c'est-à-dire qu'ils ne développent pas de SIDA. Dans ce cas le pronostic est très favorable et on peut éviter les traitements lourds. Ceci est particulièrement vrai dans le cas d'une grossesse où la présence de ces anticorps chez la mère (HIV +) semblerait conduire à une non infection du nouveau-né.

La production des compositions selon la présente invention peut être réalisée par des techniques qui sont connues, synthèse de protéine par voie chimique, synthèse d'ADN par synthèse chimique ou multiplication par amplification type PCR. Pour les protéines, celles-ci peuvent être également obtenues par voie recombinante mettant en oeuvre des synthèses appropriées.

Les exemples ci-après permettront de mettre en évidence d'autres caractéristiques et avantages de la présente invention.

Dans les figures annexées,
- les figures 1A et 1B représentent les ELISA montrant la réactivité du sérum de lapin immunisé avec R7V-KLH pour différents antigènes,
- la figure 2 représente l'ELISA montrant la réactivité de l'antisérum de lapin immunisé avec notamment la β2m,
- les figures 3A à 3D représente l'ELISA entre différents anti-sérums et des peptides sélectionnés,
- la figure 4 représente l'ELISA de R7V avec différents anticorps anti-β2m,
- la figure 5 représente l'ELISA de R7V-BSA et β2m avec des anticorps anti-β2m et des sérums de lapin,
- les figures 6 à 13 représentent des diagrammes montrant l'effet des sérums de différents patients sur la neutralisation de différents isolats du virus HIV sur MT4 et PBL.

### EXEMPLE 1

Cet exemple permet de mettre en évidence la réponse immunitaire de lapins contre des peptides sélectionnés couplés à une protéine porteuse.

L'antigène peptidique 7AA est couplé à la KLH (Keyhole Limpet Hemocyanin) et injecté aux lapins en présence d'adjuvant complet de Freund.

Les animaux sont immunisés en présence d'adjuvant complet de Freund à J0, J14, J28, J42 et des saignées d'essai sont effectuées avant l'immunisation aux jours 35, 49, 56 et 70.

Les peptides utilisés sont : RV7-KLH, S7K-KLH et F7E-KLH

Le peptide R7V (RTPKIQV) a été allongé de 2 amino-acides pour permettre le couplage. La structure utilisée comme immunogène est la RTPKIQVGY.

Les anticorps des lapins immunisés 618 ont été mesurés par ELISA, où le peptide couplé à différentes protéines porteuses a été utilisé au fond du puits (soit couplé à la KLH, à la BSA (Sérum Albumine Bovine) ou MAP (Multiple Antigenic Peptide)).

Les diagrammes représentent les résultats obtenus à 2 dilutions d100 et d1000, soit une dilution des sérums à 1/100 et 1/1000, ou à temps différents.

La méthode ELISA est appliquée de la façon suivante :

### Méthode ELISA

1) Fixation de l'antigène sur plaque 96 puits (Immulon IV-Dynatech)
   . Diluer l'antigène dans du tampon carbonate pH 9,6
      ⇒ (Ag) finale = 1 µg/ml
   . Distribuer 100 µl/puits, soit 100 ng/puits
   . Incuber 2 h à 37'C ou une nuit à 4°C (en atmosphère humide).
2) Lavages
   . 5 lavages avec une solution de PBS/Tween 20 à 0,05 %.
3) Saturation des puits
   . Distribuer 300 µl/puits d'une solution de PBS/sérum de chevel (ou boeuf) à 10 %
   . Incuber 1 h à 37°C (en atmosphère humide).
4) Lavages (identiques au point 2)
5) Incubation avec antisérums spécifiques
   . Diluer le sérum (1/50, 1/100, 1/1000) avec du PBS-10 % sérum de chevel
   . Distribuer 100 µl/puits et incuber 1 h à 37°C (en atmosphère humide).
6) Lavages (identiques au point 2)
7) Incubation avec l'anticorps second (1g de moutons anti-lg humaines couplées à la peroxidase)
   . Diluer l'anticorps second au 2/1000 dans du PBS/sérum de chevel 10%
   . Distribuer 100 µl/puits et incuber 1 h à 37°C (en atmosphère humide).
8) Lavages (identiques au point 2)
9) Révélation par l'OPD
   . Dissoudre 10 mg OPD dans 25 ml de tampon phosphocitrate (0,1 M, pH 5,5)
   . Ajouter au dernier moment 10 µl H₂O₂
   . Distribuer 100 µl/puits et incuber 30 min à l'obscurité à température ambiante (peut être lu à 405 nm)
   . Arrêter la réaction avec 50 µl H₂SO₄ 12,5 %.
10) Lecture à 492 nm.

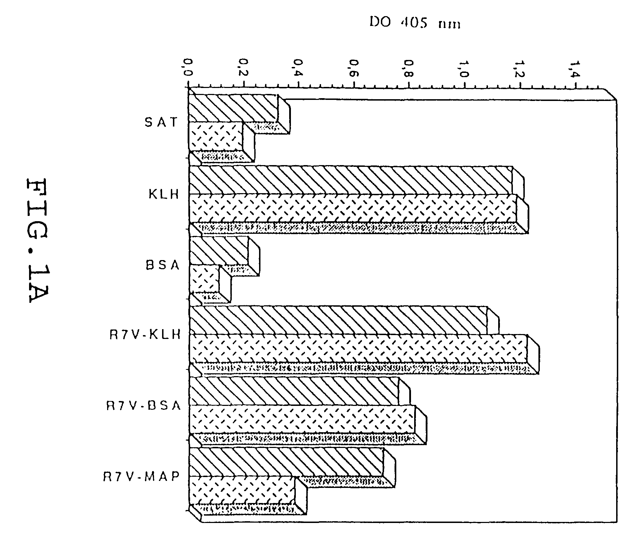

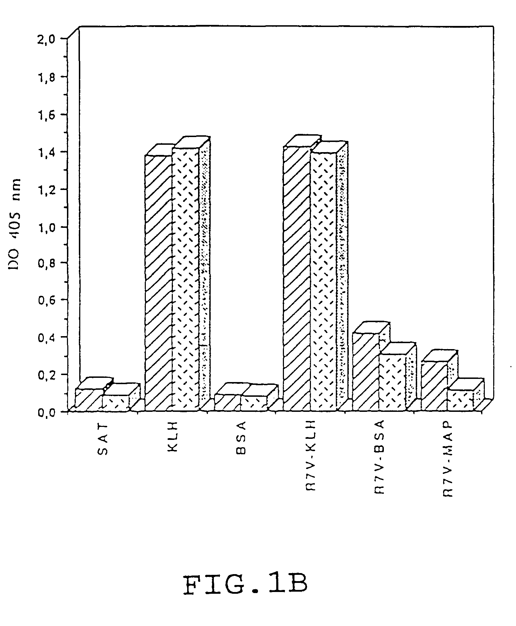

Les figures 1A et 1B montrent des résultats obtenus avec le lapin 618 immunisé avec R7V-KLH.

Une réactivité anti R7V apparaît clairement en différentiel entre R7V-BSA et BSA par rapport à R7V-KLH et KLH où la réactivité anti R7V est masquée par la réponse anti KLH du sérum. Il est à noter que la réactivité anti R7V est plus forte à J68 qu'à J132.

La spécificité de la réaction est plus grande si l'on utilise la protéine BSA.

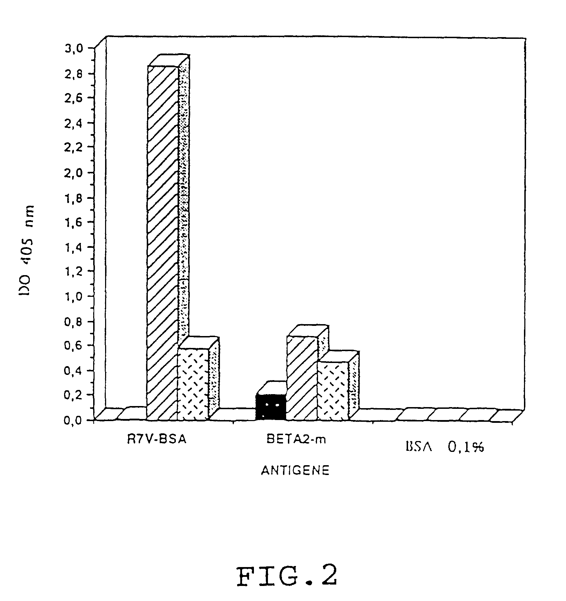

La figure 2 montre encore une bonne reconnaissance de la protéine d'origine, la β2m.

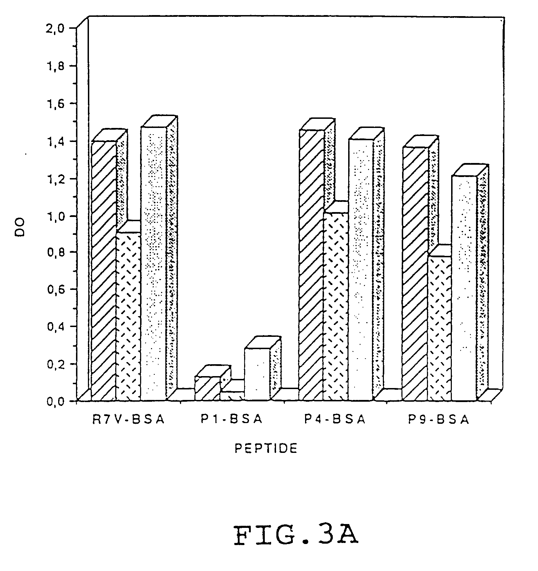

Les anti-sera de lapins immunisés démontrent une réactivité importante avec R7V-BSA ainsi qu'avec les peptides d'origine dénommés P1, P4 et P9 et qui ont été utilisés pour sélectionner R7V, même si la réactivité avec P1 est plus faible (figure 3A - 3D).

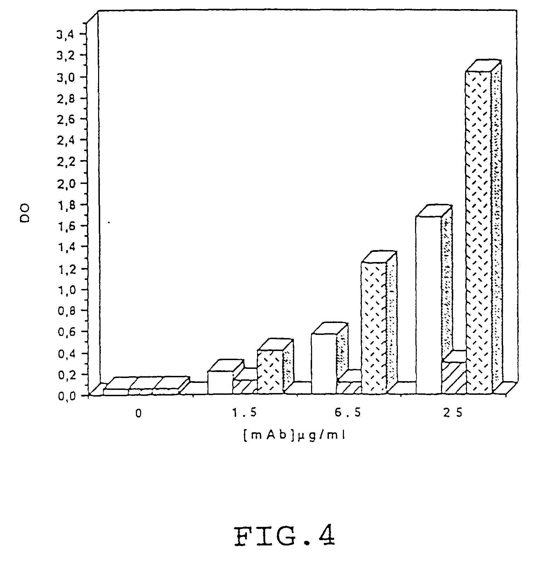

La figure 4 démontre que la reconnaissance de R7V par B1G6 et B2G2.2 est fonction de la dose et que la reconnaissance de C21.48 est moins bonne, c'est pourquoi on utilisera, de préférence, pour sélectionner des peptides équivalents les AcM B1G6 et B2G2.2.

Ces résultats démontrent que l'épitope R7V couplé à la BSA est capable de générer une bonne réponse immunitaire.

### EXEMPLE 2

### Introduction de R7V dans la boucle V3 de la gp120 de HIV-1 LAV

### Construction de provirus recombinant

L'objectif de cet exemple est d'introduire la séquence R7V dans la troisième région variable V3 de la gp120 de HIV-1 LAV.

### Méthodes

Des virus recombinants chimériques ont été construits par mutagénèse dirigée par PCR. Deux constructions basées sur la séquence R7V et HIV-1 LAV ont été obtenues, dans lesquelles sept amino-acides de la région V3 de la gp120 ont été remplacés par la séquence R7V. Les positions des séquences mutées sont montrées dans le tableau suivant :

| | | |
|---|---|---|
| HIV-1 LAV (V3) | NNNTRKSIRIQRGPGRAFVT | |
| R7V | RTPKIQV | (1) RPL |
| R7V | RTPKIQV | (2) PLG |

Le fragment EcoRI₅₂₇₈-XhoI₈₄₀₁ de HIV-1 LAV cloné dans le vecteur Bluescript a été utilisé comme matrice pour les constructions ultérieures. Dans la première étape, les fragments d'ADN flanqués des amorces contenant le site de restriction BglII à une extrémité et la séquence nucléotidique codant pour R7V à l'autre extrémité ont été synthétisés pour les constructions RPL et PLG par amplification PCR. Les oligonucléotides de mutagénèse utilisés consistaient en une amorce (+) ACACCAAAGATACAAGTTGTTACAAATAGGAAAA et une amorce (-) TTGTATCTTTGGTGTTCTCTGGATCCGGATACTTT pour la construction RPL et d'une amorce (+) CGTACACCAAAAATCCAGGTCCAGAGAGGACCA et d'une amorce (-) GATTTTTGGTGTACGCGTATTGTTGTTGGGTCT pour la construction PLG. Dans la seconde étape, deux produits de PCR pour chaque construction ont été mélangés et amplifiés en utilisant les amorces contenant les sites de restriction BgIII. Les fragments RPL et PLG ont été clivés par l'enzyme BgIII et insérés dans le vecteur Bluescript contenant le fragment EcoRI₅₂₇₈-XhoI₈₄₀₁ de HIV-1 LAV, clivé par BgIII. Outre la séquence R7V, les amorces d'amplification contenaient des modifications dans la séquence nucléotidique conduisant à l'apparition de nouveaux sites de restriction BamHI et MluI dans les constructions RPL et PLG, respectivement, sans modifications supplémentaires dans la séquence en amino-acides. Les nouveaux sites de restriction ont été utilisés pour cribler les séquences mutées. Finalement, les fragments EcoRI₅₂₇₈-XhoI₈₄₀₁ de HIV-1 LAV contenant les constructions RPL et PLG ont été insérés dans le plasmide pNL4-3 par recombinaison homologue en utilisant les sites de restriction EcoRI et Xhol. Les constructions ont été vérifiées par analyse par enzyme de restriction.

### Transfection de cellules eucarvotes

L'ADN plasmidique de 200 ml de E. coli TG1 a été extrait et purifié par le kit de midipréparation Qiagen. Les cultures semiconfluentes de cellules COS (≈ 4 x 10⁶) ont été transfectées avec 7 µg de plasmide par la technique de coprécipitation au calcium. Le jour suivant, les monocouches de cellules ont été traitées par le glycérol et mises en coculture avec une lignée cellulaire CEM ou avec des lymphocytes sanguins primaires activés par la PHA (PBL, 10⁶ cellules/ml) provenant d'un donneur sain. Les cellules CEM ou PBL ont été séparées des COS en monocouches deux jours après et cultivées séparément.

### Production de virus

1 ml de surnageant cellulaire libre obtenu à partir des cellules COS ou PBL a été ultracentrifugé et le virus sédimenté à été contrôlé par la réaction standard de la réverse transcriptase. Dans certaines expériences, 100 µl de surnageant cellulaire a été testé pour la production de la protéine p24gag.

| Transfection des cellules COS et coculture avec les CEM | | | |
|---|---|---|---|
| J. post-transf. | Activité Réverse RPL 1 | Transcriptase PLG 2 | (cpm/ml) NL 4-3 |
| 5 | 7282 | 7730 | 45838 |
| 9 | 3282 | 5302 | 326618 |
| 13 | 382 | 630 | ND |
| 16 | 200 | 300 | ND |

4 10⁶ cellules COS ont été transfectées par 7 µg de plasmide par la technique de coprécipitation au calcium. Les cellules CEM ont été alors ajoutées à raison de 4 10⁵ cellules/ml dans un volume final de 5 ml. Après deux jours de coculture les CEM en suspension ont été séparées des COS en monocouche. L'activité de la réverse transcriptase dans les surnageants de culture des CEM est donnée en cpm/ml.

| Infection des PBL | | | | |
|---|---|---|---|---|
| J. post-inf. | Activité Réverse Transcriptase (cpm/ml) | | | |
| | RPL 1 | PLG 2 | NL 4-3 | |
| 4 | 734 | 782 | 20008 | |
| 7 | 202 | 216 | | |
| 10 | 262 | 282 | | |
| 14 | 454 | 262 | | |
| 17 | 204 | 138 | | |

| | | | RPL 1 + PBL | PLG 2 + PBL 2 |
|---|---|---|---|---|
| 1 | 350 | 336 | 276 | 636 |
| 24 | 230 | 282 | 296 | 284 |
| 27 | 588 | 510 | 620 | 980 |

2,5 10⁶ PBL ont été infectées par les surnageants acellulaires du 19 décembre 1994 obtenus après transfection (tableau 1) à raison de 5000 cpm / 10⁶ PBL Au jour 17 post-infection, 2 10⁶ PBL nouvellement isolées ont été ajoutées à 2 10⁶ PBL infectées (RPL 1 + PBL, PLG 2 + PBL). L'activité de la réverse transcriptase dans les surnageants de culture est donnée en cpm/ml.

| Transfection des cellules COS et coculture avec des PBL | | | | |
|---|---|---|---|---|
| J. post-transf. | Activité Réverse Transcriptase (cpm/ml) | | | |
| | PLG 2-25 | PLG 2-30 | PLG 2-95 | NL 4-3 |
| 3 | 2500 | 8400 | 3500 | 2150 |
| 7 | 446 | 398 | 582 | 53000 |
| 10 | 174 | 336 | 306 | 74000 |
| 14 | 730 | 834 | 482 | 45778 |
| | | | | |

| J. post-transf. | | Activité R.T. (cpm/ml) | | |
|---|---|---|---|---|
| | | RPL 1 | PLG 2 | |
| 3 | | 20338 | 22000 | |
| 7 | | 682 | 418 | |
| 11 | | 552 | 466 | |

4 10⁶ cellules COS ont été transfectées par 7 µg de plasmide par la technique de coprécipitation au calcium. Les cellules PBL stimulées à la PHA ont été alors ajoutées à raison de 10⁶ cellules/ml dans un volume final de 5 ml. Après deux jours de coculture les PBL en suspension ont été séparées des COS en monocouche. L'activité de la réverse transcriptase dans les surnageants de culture des PBL est donnée en cpm/ml.

### EXEMPLE 3

Cet exemple a pour but d'utiliser les peptides sélectionnés pour détecter dans le sérum des patients des anticorps potentiellement inhibiteurs de HIV (anticorps anti β2 microglobuline) et en particulier de mettre en évidence la présence d'anticorps protecteurs dans le sérum des patients non progresseurs. Par "patients non progresseurs" ou "NP", on désigne des patients séropositifs depuis plus de 10 ans et n'ayant pas développé de SIDA, en particulier dont le taux de cellules T4 est normal.

### Matériel et Méthode

1/ Les peptides utilisés ont été synthétisés et couplés à la BSA par Néosystem (France).
2/ Les sérums des patients sont stockés à -20° ou -80° C avant leur utilisation en Elisa.
3/ Les anticorps seconds anti-Ig humaines ou de lapin ont été obtenus de Amersham (France). L'OPD provient de Sigma (France).

### ELISA avec les sérums de patients séropositifs

1/ Présence d'anticorps anti R7V dans le sérum des patients (titre 1/100 et 1/1000).
2/ Parmi les 46 sérums testés de personnes non progresseurs (pas de réplication virale en culture), 16 sérums sont positifs pour R7V (37%), 27 restent négatifs au 1/100 (63%) et 3 sérums sont indéterminables (Tableau 1).
3/ Parmi les 46 patients non progresseurs, 34 ont été testés pour la détection d'anticorps anti peptide : R7V, P1, P4, P9. 14 sérums sont positifs pour au moins un peptide (51.8%) et 13 restent négatifs au 1/100. Quatre sérums n'ont pas pu être classés positif ou négatif (Tableau 2).

**Tableau 1 :**

| ELISA R7V avec des sera NP | | |
|---|---|---|
| **NOM** | **NOMBRE** | **R7V** |
| ARA GE | 950 | Négatif |
| ARG CH | 150 | Non-déterminé |
| AUD PA | 1509 | Négatif |
| BAT AL | 134 | Négatif |
| BAR JE | 342 | Positif |
| BER AL | 704 | Positif |
| BER SE | 1337 | Négatif |
| BES LA | 287 | Positif |
| BEU PH | 5.33 | Négatif |
| BOR EM | 194 | Négatif |
| BOU NA | 5.36 | Positif |
| BRE FR | 20.2.95-5.32 | Négatif |
| CAB MI | 573 | Négatif |
| CAU BE | 167/1113 | Négatif |
| CHI OL | 353A | Négatif |
| COUDA | 1531 | Négatif |
| DIB AN | 872 | Positif |
| DUR JE | 937 | Positif |
| GAR AI | 986 | Négatif |
| GAS MA | 549 | Négatif |
| GUI JE | 60 | Positif |
| GUI PI | 26.1.95-2.9 | Négatif |
| HAN SO | 169/5.31 | Positif |
| HOL CH | 4.25 | Négatif |
| IBE JU | 6.39 | Positif |
| IMB PI | 327 | Non-déterminé |
| MAG HE | 143 | Négatif |
| MAN GU | 26.1.95-2.8 | Négatif |
| MAN RO | 89 | Positif |
| MAN XA | 730 | Négatif |
| MART DO | 1412 | Négatif |
| MAS SU | 115 | Négatif |
| MEN JO | 622/1382 | Positif |
| MON NA | 1010 | Négatif |
| NIC GE | 294 | Négatif |
| OUM NA | 1386 | Négatif |
| PAR FR | 23.1.95-1.7 | Négatif |
| MEN JO | 622/1382 | Positif |
| POI LI | 3.14 | Négatif |
| PUJ MA | 23.1.95-1.2 | Négatif |
| QUI AL | 23.1.95-1.5 | Négatif |
| RIO EM | 3.16 | Négatif |
| RIS HE | 2.10 | Négatif |
| ROY CH | 5.35 | Non-déterminé Positif |
| SAL YA | 13.3.95 | Négatif |
| SAN NA | 2.11 | Négatif |
| SAU CH | 171/4.27 | Positif |
| TEM ST | 1343 | Positif |
| VIA JE | 701 | Non-déterminé |
| ZUM AM | 333 | Positif |

**Tableau 2 :**

| ELISA des peptides avec des sera NP | | | | | | |
|---|---|---|---|---|---|---|
| **NOM** | **NOMBRE** | **POSITIF/NEGATIF** | **PEPTIDES** | | | |
| | | | R7V | P1 | P4 | P9 |
| BEU PH | 5.33 | Négatif | | | | |
| BOU NA | 5.36 | Positif | Pos. | Pos. | Pos. | Nég. |
| BRE FR | 20.2.95-5.32 | Positif | Nég. | Pos. | Pos. | Nég. |
| CIF FR | 6.38 | Négatif (?) | | | | |
| ETC MA | 6.45 | (?) | | | | |
| GEM SA | 6.40 | (?) | | | | |
| GUI JE | 60 | Positif | Pos. | Pos. | Nég. | Nég. |
| GUI PI | 26.1.95-2.9 | Négatif | | | | |
| HAN SO | 169/5.31 | Positif | Pos. | Pos. | Nég. | Pos. |
| HOL CH | 4.25 | Négatif | | | | |
| IBE JU | 6.39 | Positif | Pos. | Pos. | Nég. | Nég. |
| LED DO | 4.23 | Positif | Nég. | Pos. | Nég. | Nég. |
| MAN GU | 26.1.95-2.8 | Négatif | | | | |
| MEN JO | 622/1382/6.4 3 | Positif | Pos. | Pos. | Pos. | Pos. |
| MOR JE | 5.37 | Positif | Nég. | Nég. | Pos. | Nég. |
| PAR FR | 23.1.95-1.7 | Négatif | | | | |
| PAT MA | 166 | Positif | Nég. | Nég. | Pos. | Nég. |
| PIC CH | 2.12 | (?) | | | | |
| POI LI | 3.14 | Négatif | | | | |
| PUJ MA | 23.1.95-1.2 | Négatif | | | | |
| QUI AL | 23.1.95-1.5 | Négatif | | | | |
| RIO EM | 3.16 | Négatif | | | | |
| RIS HE | 2.10 | Négatif | | | | |
| ROY CH | 5.35 | Positif | Pos.(?) | Pos. | Nég. | Nég. |
| SAL YA | 13/3.95 | Négatif | | | | |
| SAN NA | 2.11 | Négatif | | | | |
| SAP MA | 4.21 | (?) | | | | |
| SAU CH | 171/4.27 | Positif | Pos. Pos.(?) | Pos. | | Pos. |
| SEN AN | 4.28 | Positif | Nég. | Pos. | Nég. | Nég. |
| TEM ST | 5.34 | Positif (?) | Pos. | (?) | (?) | (?) |
| ZUM AM | 333 | Positif | Pos. | (?) | (?) | (?) |
| (?) Non-déterminé | | | | | | |

### EXEMPLE 4

Les essais suivants ont permis de mettre en évidence chez les patients non progresseurs des anticorps neutralisant différents isolats de HIV, notamment BRU et NDK, les mêmes patients présentant des anticorps anti-R7V. Ceci permet de montrer une bonne corrélation entre le caractère neutralisant et protecteur à l'encontre de HIV des anticorps générés par les traitements selon l'invention.

### Matériel et méthodes

### Culture des cellules MT4

Les cellules MT4 sont des cellules immortalisées (CD4 +) très sensibles à l'effet cytopathogène de HIV-1, dérivées à l'origine d'une leucémie T chez l'adulte. Les cellules sont cultivées en présence de milieu RPMI supplémenté avec 10 % de sérum de veau foetal, 1 % de glutamine et 1 % d'antibiotique.

### Culture des PBL

Les lymphocytes sont stimulés pendant 3 jours avec de la phyto-hémagglutinine P (PHA P) dans du milieu RPMI complet comprenant 10 % de sérum de veau foetal, 1 % de glutamine, 1 % d'antibiotique, 2 µg/ml de polybren, 20 Ul/ml d'Interleukine 2 (IL-2). Les cellules sont ensuite lavées et cultivées à raison de 10⁶ cellules/ml dans du milieu RPMI complet.

### Tests de neutralisation

Les sérums sont décomplémentés et filtrés avant leur utilisation dans les tests.

### Neutralisation sur MT4

Les sérums sont dilués en plaques 24 puits (Costar) dans un volume total de 0,8 ml. les virus HIV-1 BRU (100 µl d'une dilution 10⁻¹ de la solution stock) ou HIV-1 NDK (100 µl d'une dilution 10⁻³ de la solution stock) sont ajoutés et le mélange est incubé pendant 1 h 30 à 37°C et 5 % CO2. Les cellules sont ensuite distribuées à raison de 200 µl/puits et 1, 5 10⁶ cellules/ml. Trois jours après l'infection, les cultures sont diluées (1/3) avec du milieu RPMI 10 %. L'infection des cellules par les virus HIV-1 est suivie tous les jours sous microscope par l'observation de la formation de syncitia (cellules géantes multinuclées). La neutralisation des virus par les différents sérums est définie par l'absence (-) de syncitia ou très peu de syncitia (+/-) en comparaison de la formation de syncitia induite par les HIV prototypes (+).

### Neutralisation sur PBL

Les sérums (50 µl) sont mélangés avec les virus HIV-1 BRU (50 µl d'une dilution 2 10⁻¹ de la solution stock) dans des plaques 96 puits et placés pendant 1 h 30 à 37°C et 5 % CO2. Le mélange est alors ajouté à 10⁶ PBL dans des plaques 24 puits (Costar) et la culture est maintenue pendant 3 jours à 37°C et 5 % CO2. Les cellules sont ensuite lavées et cultivées à raison de 10⁶ cellules/ml dans des flacons de culture de 25 cm². La production de virus est suivie tous les 3 ou 4 jours par le dosage de l'activité enzymatique de la "Reverse Transcriptase".

### Dosage de l'activité "Reverse Transcriptase" (RT)

Un ml de surnageant de culture centrifugé (1500 rpm), TA, 10 min) est concentré 100 fois par ultracentrifugation (95000 rpm, 4°C, 5 min) sur un rotor TL 100 (Beckman). Le culot obtenu est repris dans 10 µl de tampon NTE-0,1 % Triton X100. La réaction enzymatique est réalisée dans 50 µl du mélange réactionnel suivant : Tris 50 mM pH 7,8 ; MgCl₂ 20 mM ; KCI 20 mM : Dithiothreitol 2 mM; Oligo dT 12-18 0,25 OD/ml : poly rA 0,25 OD/ml et 2,5 µCi de ³HdTTP. Après 1 h d'incubation à 37°C, les produits de la réaction sont précipités avec de l'acide trichoracétique 20 %, filtrés sur des membranes Millipore et la radioactivité β est mesurée. Les résultats sont exprimés en CMP/ml.

### Rapport des experiences de neutralisation

Des anticorps dirigés contre le peptide R7V ont été mis en évidence dans les sérums de patients HIV + au moyen d'un ELISA spécifique mis au point par la Demanderesse. On a cherché dans ces sérums l'existence d'une activité neutralisantes dirigée contre les deux souches de virus prototype HIV-1 BRU et NDK. Deux tests de neutralisation ont été réalisés, l'un sur une lignée cellulaire MT4 (suivi de la formation de syncitia) et l'autre sur des lymphocytes du sang périphérique sains, PBL (suivi de l'activité enzymatique "Reverse Transcriptase").

### Résultats obtenus sur MT4

Parmi les 13 patients testés, une activité sérique neutralisante a été mise en évidence pour 6 d'entre eux (Tableaux 3 à 5) :
- Deux sérums neutralisent HIV-1 NDK :
   ZUM AM (ELISA positif)
   COC PH (ELISA négatif)
- Deux sérums neutralisent HIV-1 BRU :
   MEC EV (ELISA positif)
   OUA VE (ELISA négatif)
- Deux sérums neutralisent HIV-1 BRU et HIV-1 NDK :
   SAU CH (ELISA positif)
   BUB JE (ELISA positif)

### Résultats obtenus sur PBL

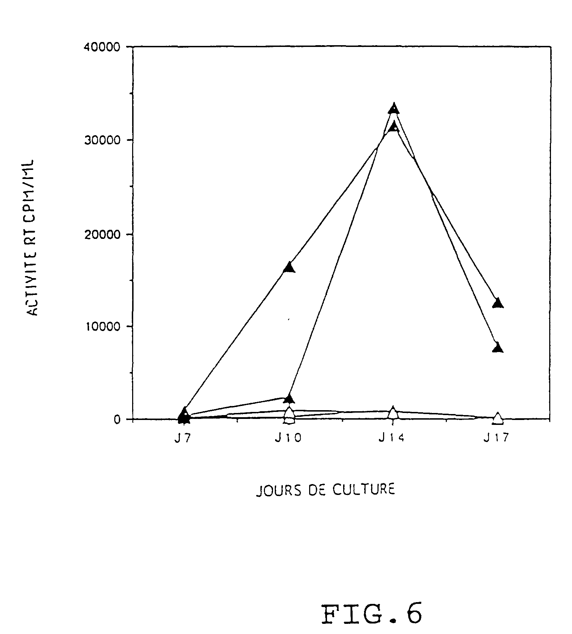

L'expérience a été réalisée avec les sérums de MEC EV, SAU CH et BUB JE (1/50) ainsi qu'avec un sérum de séronégatif. Aucune activité neutralisante n'a été détectée pour le sérum SAU CH ainsi que pour le sérum séronégatif. En revanche, une activité neutralisante contre les deux virus prototypes HIV-1 BRU et NDK a été mise en évidence pour les sérums MEC EV et BUB JE (figures 6 à 13).

### EXEMPLE 5

### Méthode permettant de mettre en évidence des peptides équivalents

### Effet de peptides sélectionnés sur la neutralisation de HIV-1 NDK par l'anticorps monoclonal anti-β2 B1G6

### Protocole

Les peptides à une concentration de 100 µg/ml ou 50 µg/ml (40 µl ou 20 µl de la solution mère à 5 mg/ml) sont préincubés avec 5 µg/ml de B1G6 (10 µl d'une solution mère à 1 mg/ml) dans un volume total de 110 µl pendant 2 heures, dans des tubes au bain-marie à 37°C, sous agitation douce. Puis HIV-1 NDK est ajouté (100 µl d'une dilution à 2.10⁻⁴ d'une solution mère et les tubes sont incubés pendant 1 heure à 37°C au bain-marie. Les tubes sont ensuite séparés en deux et chaque 100 µl est ajouté à 10⁶ PBL sur une plaque à 24 puits. Les cellules sont cultivées pendant 3 jours à 37°C sous une atmosphère à 5 % de CO₂. Au jour 3, les cellules sont lavées, mises en culture et propagées pendant au moins 20-25 jours dans un ballon de 25 cm³. La production de virus est suivie tous les 3 ou 4 jours, par dosage de la réverse transcriptase (RT).

### Résultats

Les peptides R7V et F7E peuvent annuler l'effet de neutralisation de l'anticorps monoclonal B1G6 sur la production de HIV-1 NDK par les PBL. La séquence du peptide R7V a été modifiée et parmi les 6 nouveaux peptides (185, 186, 187, 188, 189, 190), 3 ont perdu l'effet d'annulation du R7V : les peptides 185, 189 et 190.

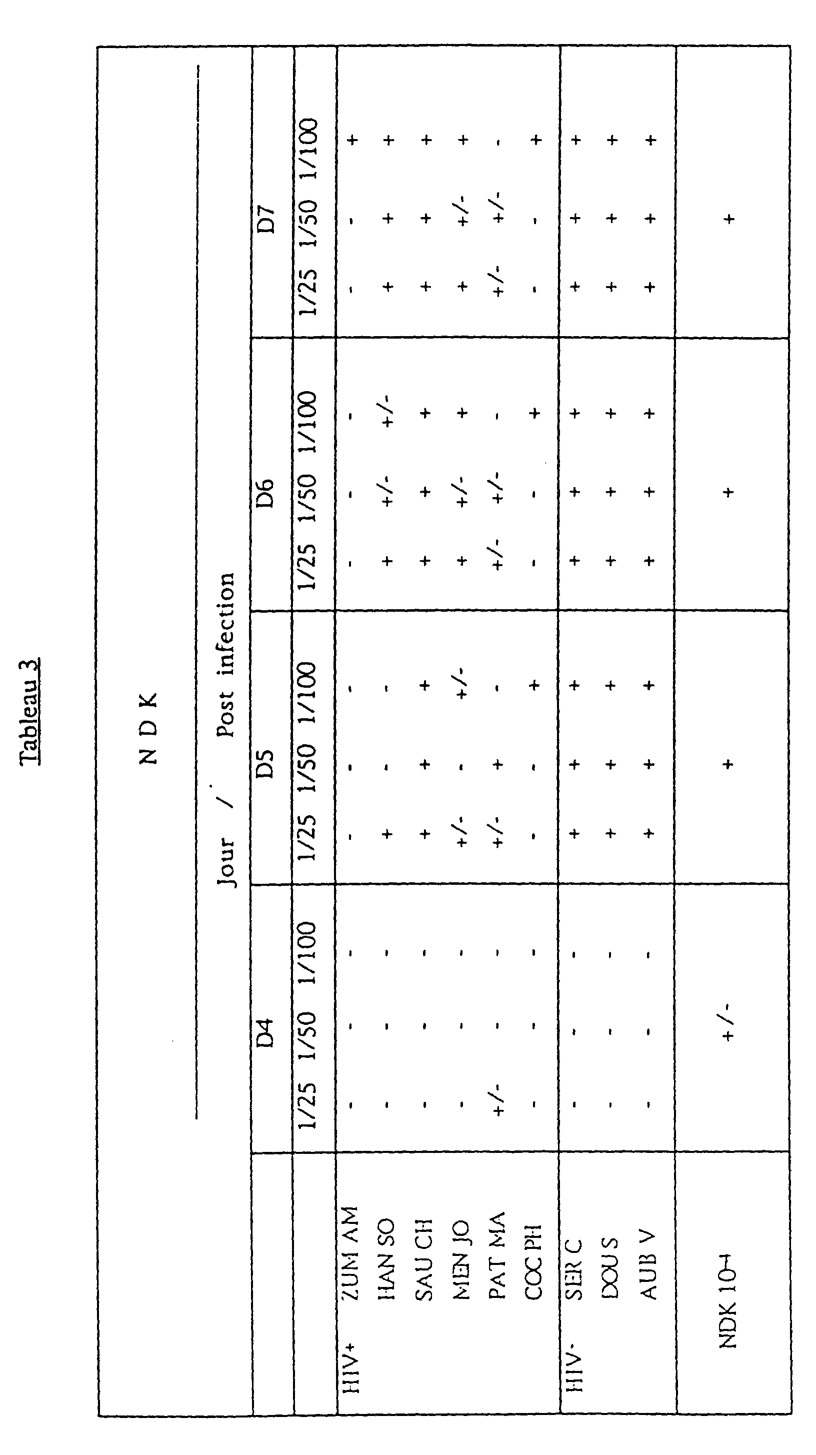

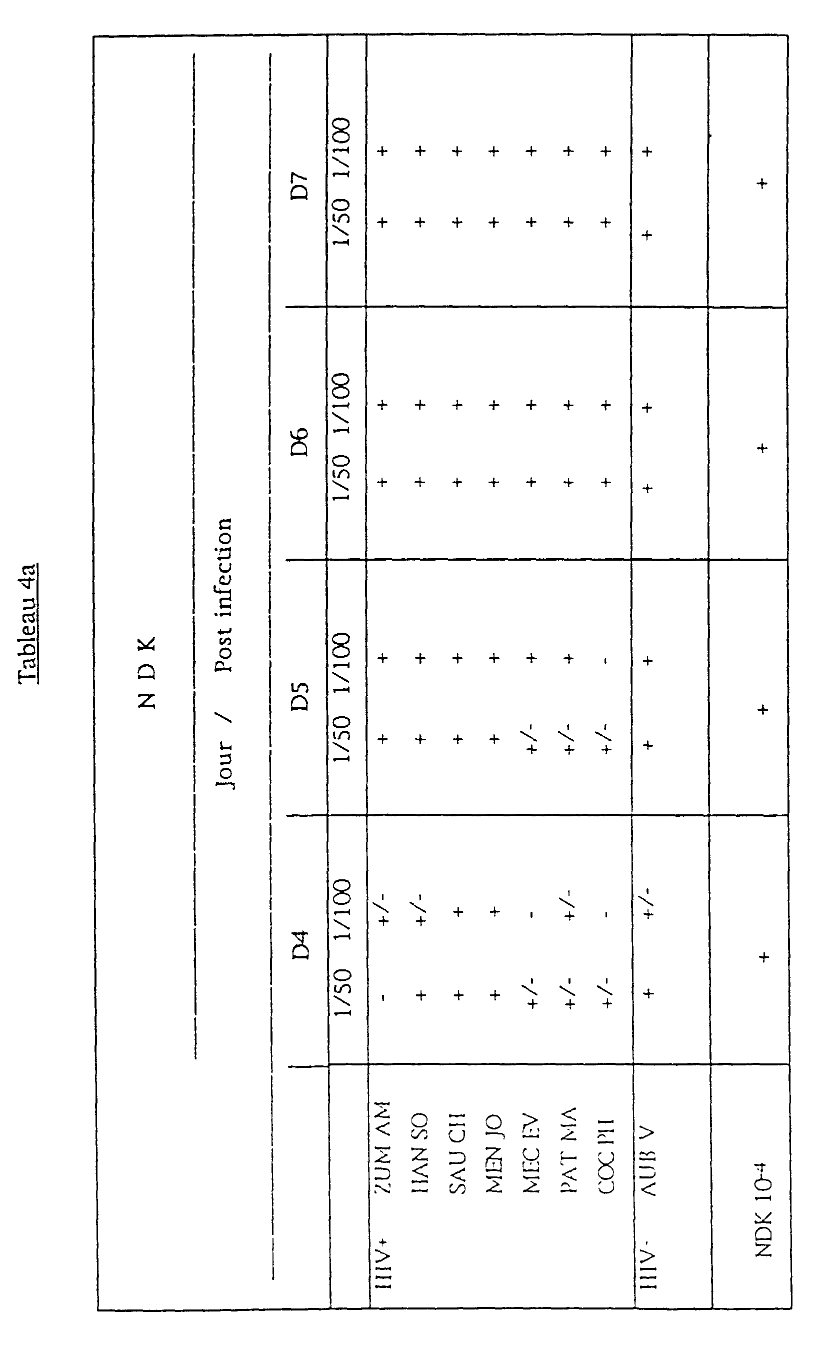

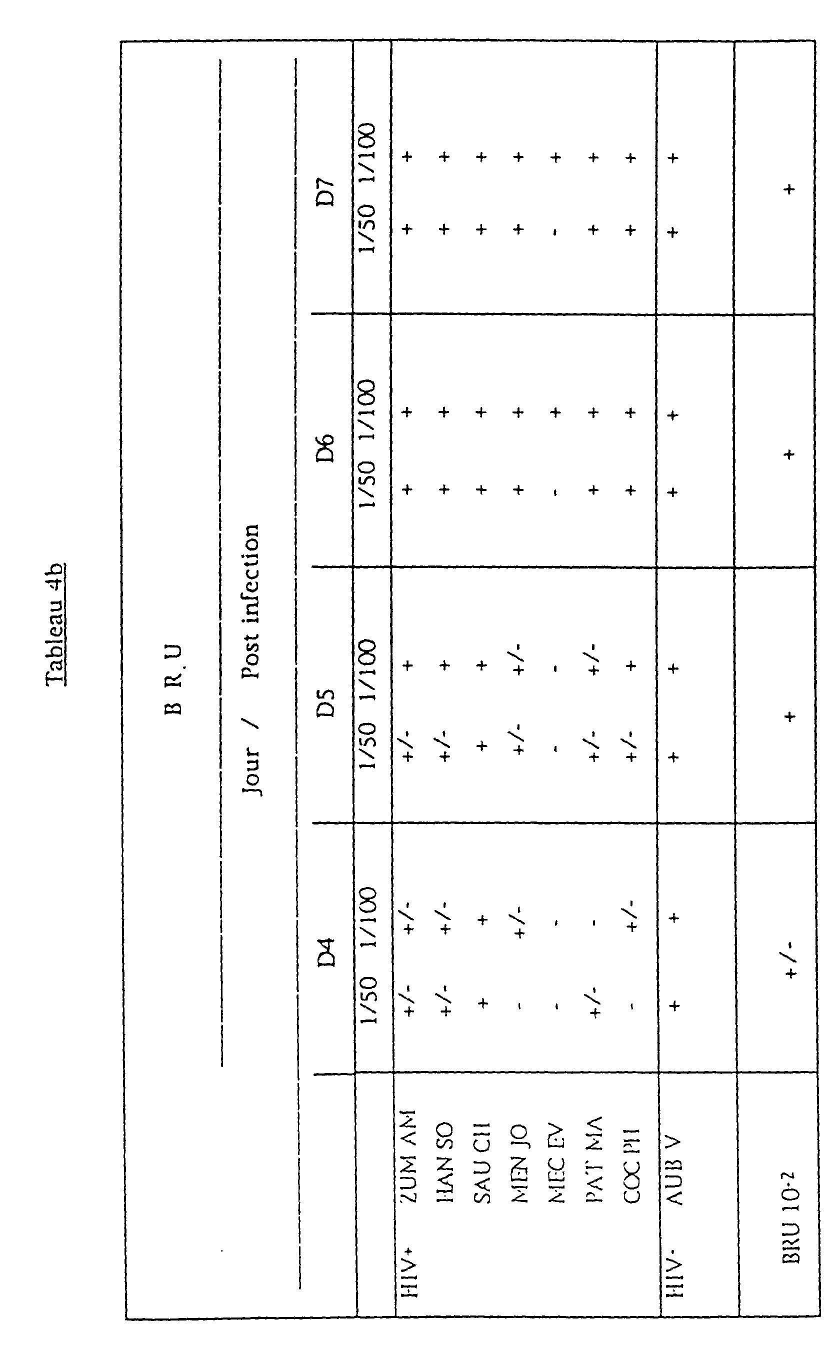

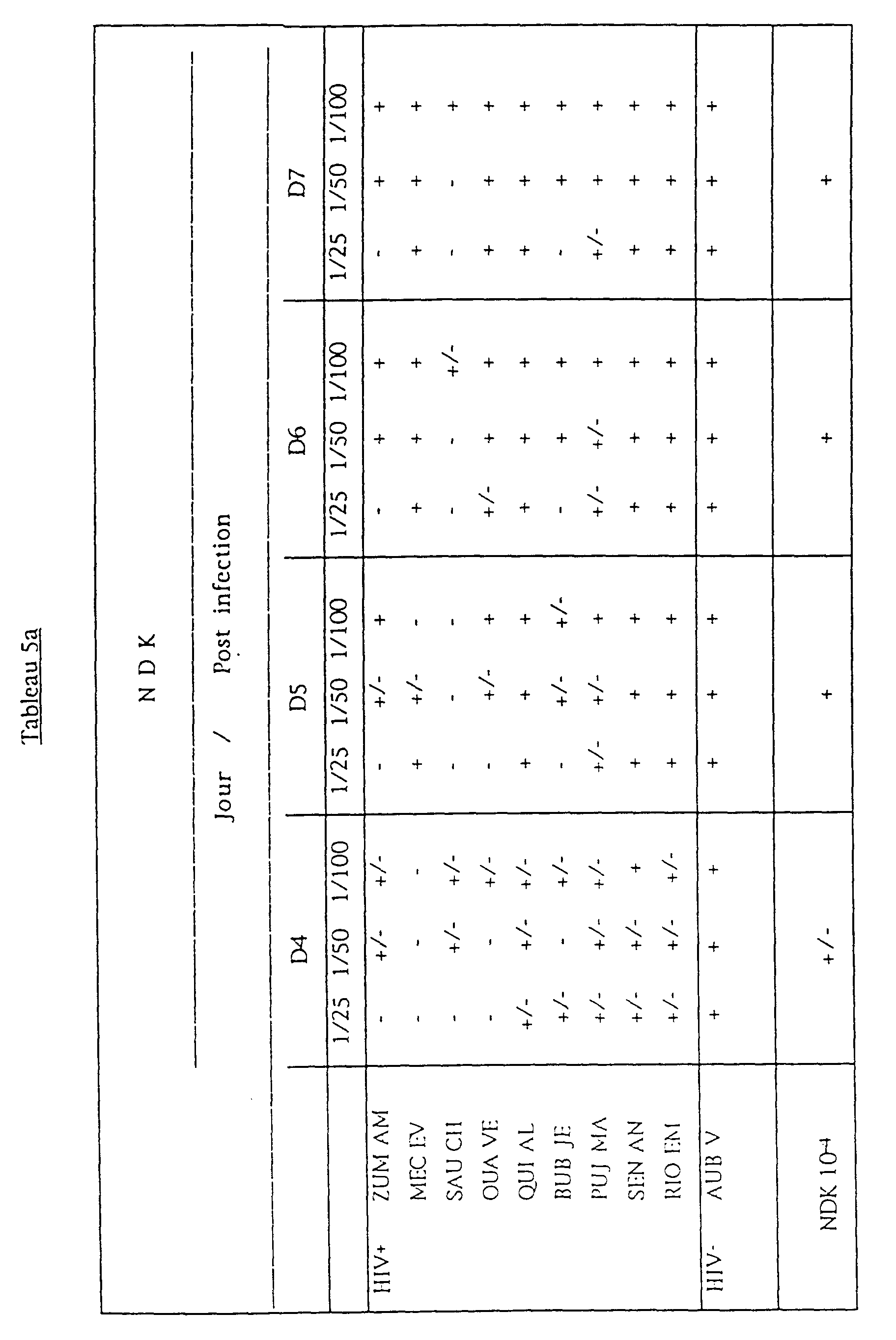

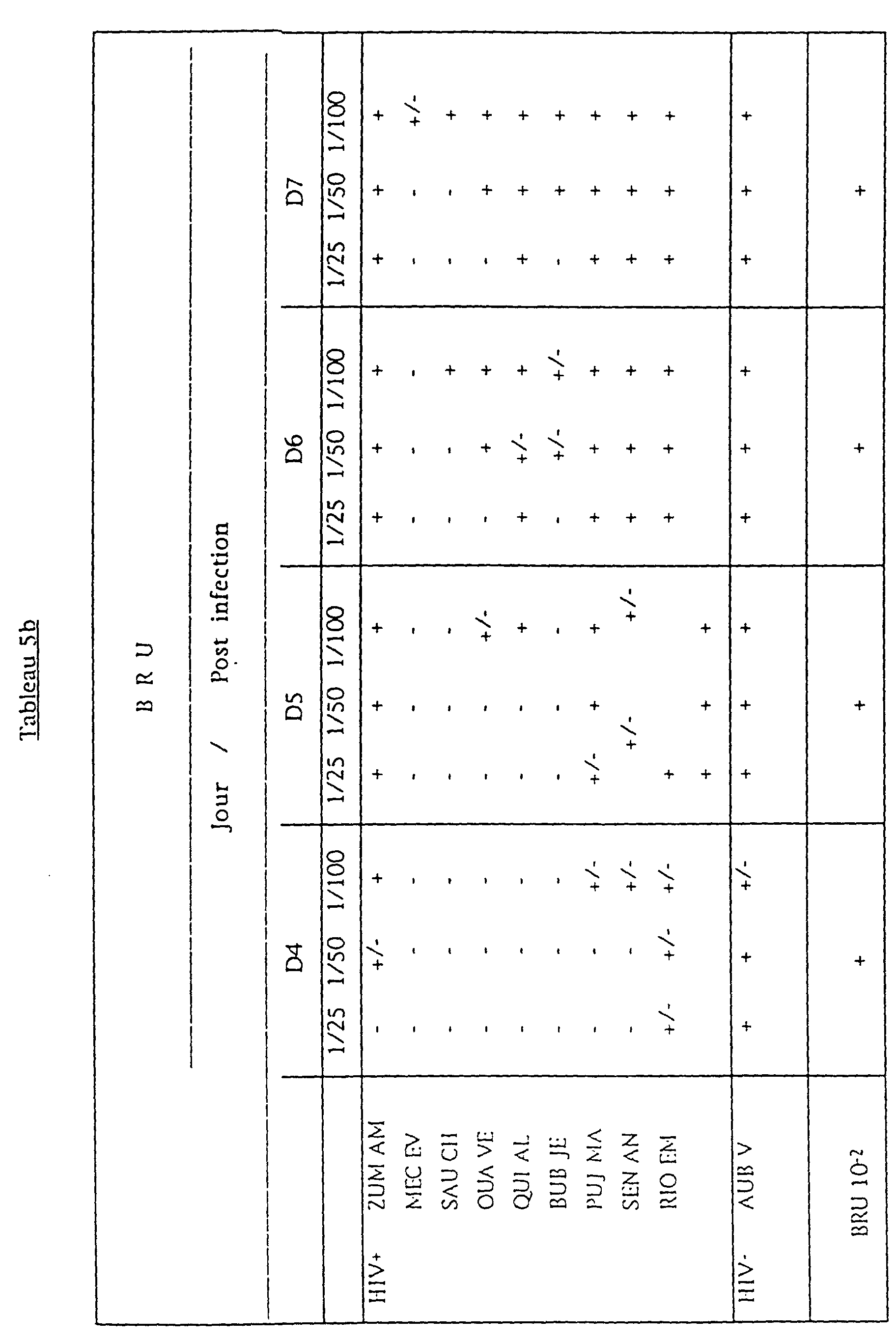

### EXEMPLE 6

### Corrélation entre la présence d'anticorps anti-R7V et la progression de la maladie

Des échantillons de sérums de 90 patients infectés par le HIV sont utilisés. Ils se répartissent comme suit : 28 patients asymptomatiques depuis plus de 3 ans, 24 survivants à long terme et 38 patients atteints du Sida. Un groupe de contrôle constitué de 69 donneurs volontaires séronégatifs a été obtenu à partir de la banque du sang.

Le comptage des lymphocytes a été effectué par immunofluorescence indirecte et analysé par Epic Profile (Coultronics, Margency, France). Les taux sériques de β2m ont été mesurés par immunodiffusion (El Nanorid Kit). Les taux d'antigène p24 ont été testés par le Kit de détection p24 de Coulter (Coultronics, Margency, France).

Les concentrations sériques en anticorps anti-R7V sont détectées par ELISA. Les résultats sont exprimés en concentration d'équivalent d'anticorps monoclonaux B1G6 en *µ*g/ml.

### Essai de neutralisation

Les sérums humains sont décomplémentés et dilués jusqu'à 200 *µ*g/ml ou 100 *µ*g/ml d'équivalent B1G6. 50 *µ*l de HIV contenant 100 TCID₅₀ sont préincubés avec 50 *µ*l de sérum dilué (volume total 100 *µ*l) dans une plaque à 96 puits à 37° C et 5% de CO₂ pendant 90 min. Le mélange réactionnel contenant les virus et le sérum est dilué deux fois après addition de 8x10⁴ cellules MT4 (dilution finale des sérums de 1/120 à 1/20) et trois fois à nouveau trois jours après l'infection. L'effet fusogénique du HIV dans les lignées MT4, c'est-à-dire la formation de syncytia comme indicateur de l'infection par le virus, est suivi pendant 7 jours dans les puîts de culture. L'activité Reverse Transcriptase est mesurée dans 400 *µ*l de surnageant dépourvu de cellule, 7 jours après l'infection.

La valeur moyenne des taux d'anticorps anti-R7V est calculée pour chaque patient et pour chaque groupe. Les sérums de personnes infectées par le HIV contiennent des anticorps anti-R7V et les sérums HIV séropositifs montrent des concentrations significativement plus élevées en anticorps anti-R7V que celles des sérums séronégatifs. Les taux d'anticorps anti-R7V exprimés en équivalent B1G6 s'échelonnent de 35 à 2558 *µ*g/ml (n = 90) et de 27 à 1790 *µ*g/ml (n = 69), respectivement dans les groupes infectés par HIV et dans les groupes non infectés par HIV.

On a ensuite classé le groupe des patients HIV en trois catégories selon leur statut clinique : groupe des non-progresseurs (NP) constitué des patients séropositifs pour HIV depuis une longue période et suivis au Laboratoire depuis plus de 3 ans sans symptômes du Sida ; le groupe des survivants à long terme (LTS) est constitué des personnes présentant le Sida depuis une longue période et finalement, un groupe progresseur est constitué des personnes atteintes du Sida avec un mauvais pronostic.

Les anticorps anti-R7V sont significativement augmentés dans le groupe asymtomatique (de 91 à 2558 *µ*g/ml) par rapport au groupe progresseur (de 35 à 630 *µ*g/ml) (p = 0,001) alors qu'on n'observe pas de différence significative par rapport au groupe LTS ( de 59 à 1864 *µ*g/ml). De même le groupe LTS présente des taux d'anti-R7V plus élevé que le groupe progresseur (p = 0,004). Comparé aux sujets sains, il n'existe pas de différence du taux d'anticorps anti-R7V dans le groupe progresseur.

Dans le groupe progresseur, une distinction nette peut être faite en fonction du taux d'anticorps anti-R7V entre les sujets qui décèdent peu de temps après leur dernière visite au Laboratoire (de 35 à 508 *µ*g/ml, n = 23) et ceux encore vivants mais malades (de 77 à 586 *µ*g/ml, n = 14) (p < 0,03).

Une étude suivie longitudinale n'a pas pu établir de corrélation entre les taux d'anticorps anti-R7V et d'autres paramètres biologiques comme la numération totale des lymphocytes, les cellules CD4, CD8, la p24 et la β2m circulant.

Il apparaît que le taux de R7V est stable dans le temps chez les patients NP, alors qu'il fluctue chez les patients LTS.

Afin de lier le test ELISA avec une activité biologique du sérum du patient, un test de neutralisation a été effectué avec deux virus non apparentés, la souche HIV-1 LAV et la souche hautement cytopathogène HIV-1 NDK, sur des cellules MT4 indicateur. Les dilutions du sérum ont été ajustées afin d'obtenir 5 *µ*g d'équivalent B1G6 dans le mélange de neutralisation. Cette concentration avait été définie comme optimum pour la neutralisation de l'infection par les anticorps B1G6. Comme on le voit dans le tableau 5, 17 des 18 sera sélectionnés empêchent l'infection de cellules MT4 aussi bien par NDK que par LAV. Pour parvenir à 5 *µ*g d'équivalent B1G6 en culture, 13 des 18 sera testés nécessitaient une dilution inférieure à 1/50. Afin d'éliminer des activités non spécifiques dues à d'éventuels composants sériques, ces sérums à faible taux en équivalent B1G6 ont été dilués au 1/100 et utilisés dans l'essai de neutralisation. La quantité d'équivalent B1G6 en culture était alors inférieure à 5 *µ*g (de 2,5 *µ*g/ml à 0,3 *µ*g/ml). Neuf des 14 sérums (64%) neutralisaient encore les deux souches HIV, LAV et NDK, à une dilution au 1/100. Trois sérums de donneurs sains utilisés comme contrôles, ne montrent aucune activité neutralisante.

**Tableau 5**

| Dilutions du sérum à 5 µg d'équivalent B1G6 dans l'essai | Nombre de sérums qui neutralisent les deux souches de HIV/sera total testé |
|---|---|
| dilution ≥ 1/50 | 5/5 |
| 1/50 > dilution ≥ 1/20 | 10/11 |
| dilution < 1/20 | 2/2 |
| TOTAL | 17/18 |

### LISTE DE SEQUENCES

(1) INFORMATIONS GENERALES:
   (i) DEPOSANT:
      (A) NOM: INSTITUT NATIONAL DE LA SANTE ET DE LA RECHERCHE MEDICALE (INSERM)
      (B) RUE: 101, RUE DE TOLBIAC
      (C) VILLE: PARIS
      (D) ETAT OU PROVINCE: 75013
      (E) PAYS: FRANCE
      (F) CODE POSTAL: 75013
   (ii) TITRE DE L'INVENTION: VACCIN CONTRE DES AGENTS INFECTIEUX AYANT UNE PHASE INTRACELLULAIRE, COMPOSITION POUR LE TRAITEMENT DE LA PREVENTION DES INFECTIONS A HIV, ANTICORPS ET PROCEDE DE DIAGNOSTIC
   (iii) NOMBRE DE SEQUENCES: 22
   (iv) FORME DECHIFFRABLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.30 (OEB)
(2) INFORMATIONS POUR LA SEQ ID NO: 1:
   (i) CARACTERISTIQUES DE LA SEQUENCE:
      (A) LONGUEUR: 15 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 1:
   Ile Gln Arg Thr Pro Lys Ile Gln Val Tyr Ser Arg His Pro Ala
   1               5                   10                  15
(2) INFORMATIONS POUR LA SEQ ID NO: 2:
   (i) CARACTERISTIQUES DE LA SEQUENCE:
      (A) LONGUEUR: 15 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 2:
   Phe His Pro Ser Asp Ile Glu Val Asp Leu Leu Lys Asp Gly Glu
   1 5 10 15
(2) INFORMATIONS POUR LA SEQ ID NO: 3:
   (i) CARACTERISTIQUES DE LA SEQUENCE:
      (A) LONGUEUR: 15 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 3:
   Ala Cys Arg Val Asn His Val Thr Leu Ser Gln Pro Lys Ile Val
   1 5 10 15
(2) INFORMATIONS POUR LA SEQ ID NO: 4:
   (i) CARACTERISTIQUES DE LA SEQUENCE:
      (A) LONGUEUR: 7 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 4:
   Arg Thr Pro Lys Ile Gln Val
   1 5
(2) INFORMATIONS POUR LA SEQ ID NO: 5:
   (i) CARACTERISTIQUES DE LA SEQUENCE:
      (A) LONGUEUR: 7 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 5:
   Ser Gln Pro Lys Ile Val Lys
   1 5
(2) INFORMATIONS POUR LA SEQ ID NO: 6:
   (i) CARACTERISTIQUES DE LA SEQUENCE:
      (A) LONGUEUR: 7 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 6:
   Phe His Pro Ser Asp Ile Glu
   1 5
(2) INFORMATIONS POUR LA SEQ ID NO: 7:
   (i) CARACTERISTIQUES DE LA SEQUENCE:
      (A) LONGUEUR: 10 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 7:
   Thr Leu Ser Arg Thr Pro Lys Ile Gln Val
   1 5 10
(2) INFORMATIONS POUR LA SEQ ID NO: 8:
   (i) CARACTERISTIQUES DE LA SEQUENCE:
      (A) LONGUEUR: 10 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 8:
   Ile Tyr Leu Thr Gln Pro Lys Ile Lys Val
   1 5 10
(2) INFORMATIONS POUR LA SEQ ID NO: 9:
   (i) CARACTERISTIQUES DE LA SEQUENCE:
      (A) LONGUEUR: 10 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 9:
   Ile Gln Arg Thr Pro Lys Ile Gln Val Tyr
   1 5 10
(2) INFORMATIONS POUR LA SEQ ID NO: 10:
   (i) CARACTERISTIQUES DE LA SEQUENCE:
      (A) LONGUEUR: 10 acides aminés
      (B) TYPE: acide amin,
      (C) NOMBRE DE BRéNS: simple
      (D) CONFIGURATION: linéaire
   (ii) TYPE DE MOLECULE: peptide
   (xi) DESCRIPTION DE LA SEQUENCE: SEQ ID NO: 10:
   Thr Leu Ser Gln Pro Lys Ile Val Lys Asn
   1 5 10
(2) INFORMATIONS POUR LA SEQ ID NO: 11:
   (i) CARACTERISTIQUES DE LA SEQUENCE:
      (A) LONGUEUR: 10 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 11:
   Ile Gln Arg Thr Pro Gln Ile Val Lys Trp
   1 5 10
(2) INFORMATIONS POUR LA SEQ ID NO: 12:
   (i) CARACTERISTIQUES DE LA SEQUENCE:
      (A) LONGUEUR: 10 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 12:
   Ile Gln Arg Thr Pro Asn Ile Val Lys Trp
   1 5 10
(2) INFORMATIONS POUR LA SEQ ID NO: 13:
   (i) CARACTERISTIQUES DE LA SEQUENCE:
      (A) LONGUEUR: 8 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 13:
   Cys Tyr Asn Pro Ser Asp Ile Glu
   1 5
(2) INFORMATIONS POUR LA SEQ ID NO: 14:
   (i) CARACTERISTIQUES DE LA SEQUENCE:
      (A) LONGUEUR: 7 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 14:
   Tyr Cys Asn Pro Glu Ser Thr
   1 5
(2) INFORMATIONS POUR LA SEQ ID NO: 15:
   (i) CARACTERISTIQUES DE LA SEQUENCE:
      (A) LONGUEUR: 8 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 15:
   Asn Phe Leu Asn Cys Tyr Val Ser
   1 5
(2) INFORMATIONS POUR LA SEQ ID NO: 16:
   (i) CARACTERISTIQUES DE LA SEQUENCE:
      (A) LONGUEUR: 9 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 16:
   Leu Asn Cys Tyr Val Ser Pro Ser Asp
   1 5
(2) INFORMATIONS POUR LA SEQ ID NO: 17:
   (i) CARACTERISTIQUES DE LA SEQUENCE:
      (A) LONGUEUR: 7 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 17:
   Lys Thr Pro Gln Ile Gln Val
   1 5
(2) INFORMATIONS POUR LA SEQ ID NO: 18:
   (i) CARACTERISTIQUES DE LA SEQUENCE:
      (A) LONGUEUR: 7 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 18:
   Phe His Pro Pro Gln Ile Glu
   1 5
(2) INFORMATIONS POUR LA SEQ ID NO: 19:
   (i) CARACTERISTIQUES DE LA SEQUENCE:
      (A) LONGUEUR: 7 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 19:
   Phe His Pro Pro His Ile Glu
   1 5
(2) INFORMATIONS POUR LA SEQ ID NO: 20:
   (i) CARACTERISTIQUES DE LA SEQUENCE:
      (A) LONGUEUR: 7 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 20:
   Ala Glu Pro Lys Thr Val Tyr
   1 5
(2) INFORMATIONS POUR LA SEQ ID NO: 21:
   (i) CARACTERISTIQUES DE LA SEQUENCE:
      (A) LONGUEUR: 7 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 21:
   Ser Gln Pro Lys Thr Val Tyr
   1 5
(2) INFORMATIONS POUR LA SEQ ID NO: 22:
   (i) CARACTERISTIQUES DE LA SEQUENCE:
      (A) LONGUEUR: 10 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 22:

### LEGENDE DES FIGURES

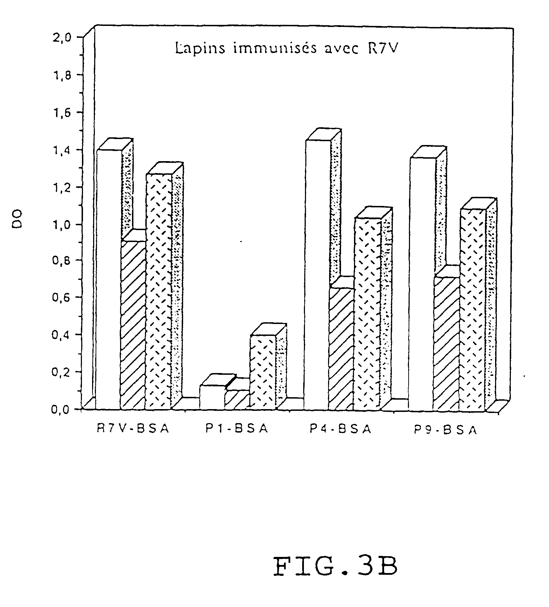

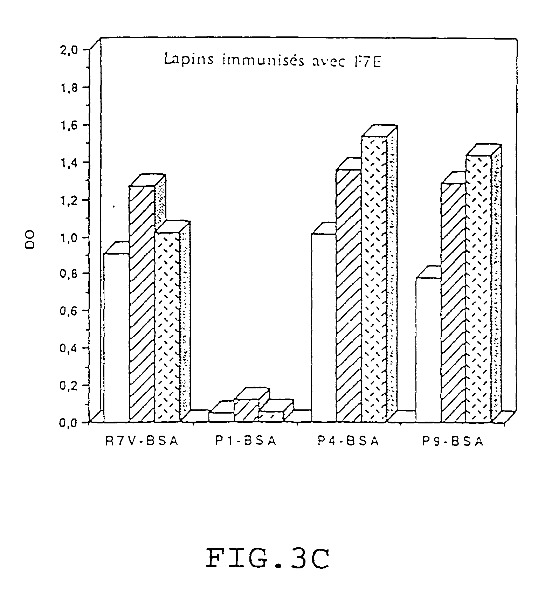

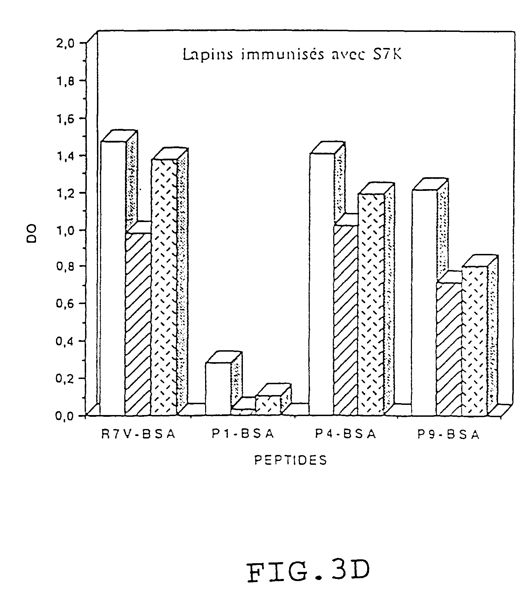

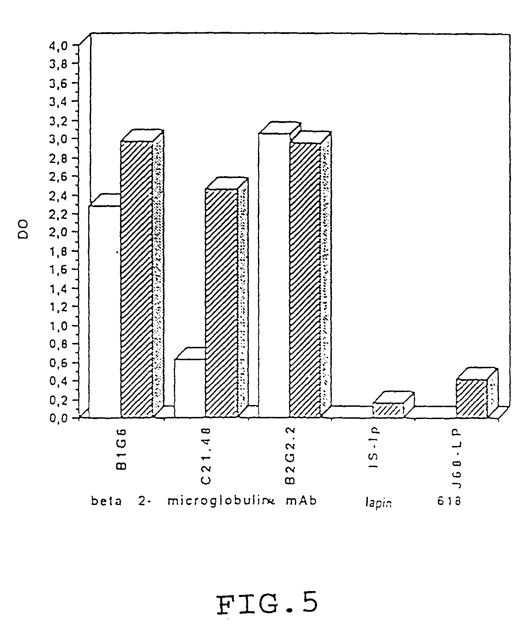

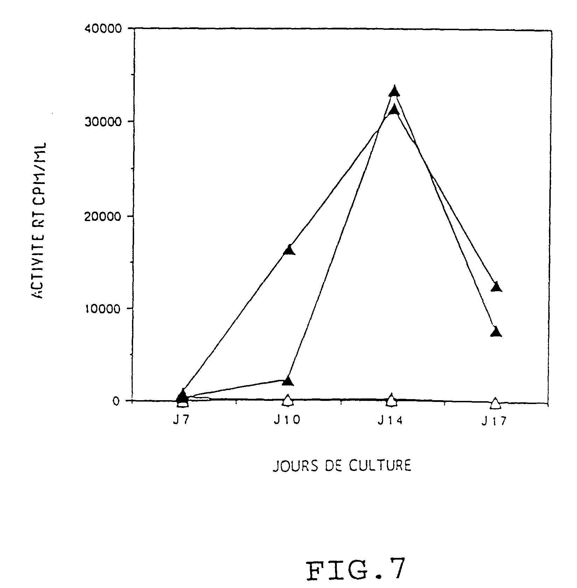

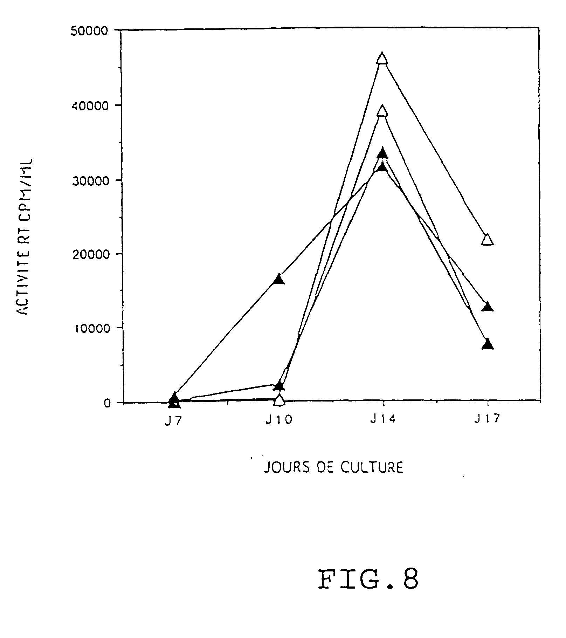

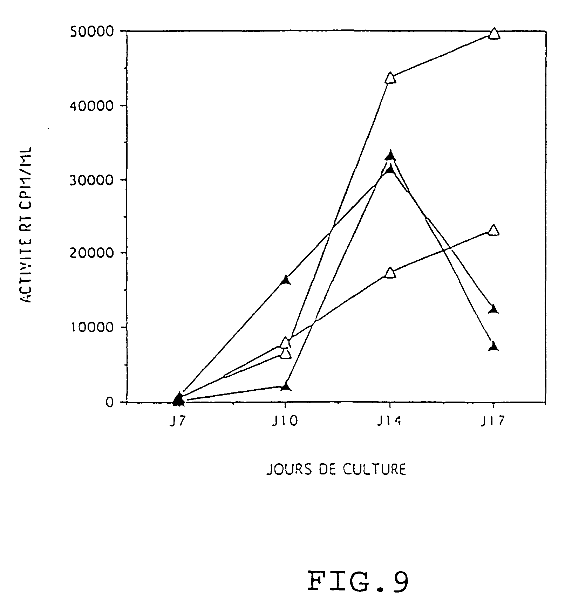

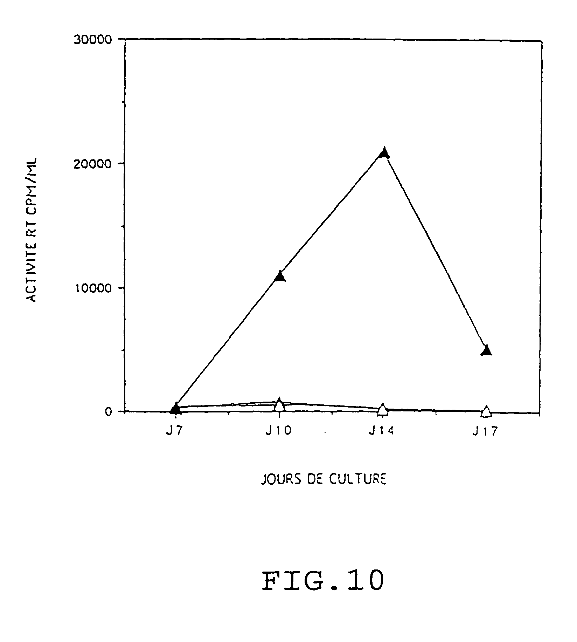

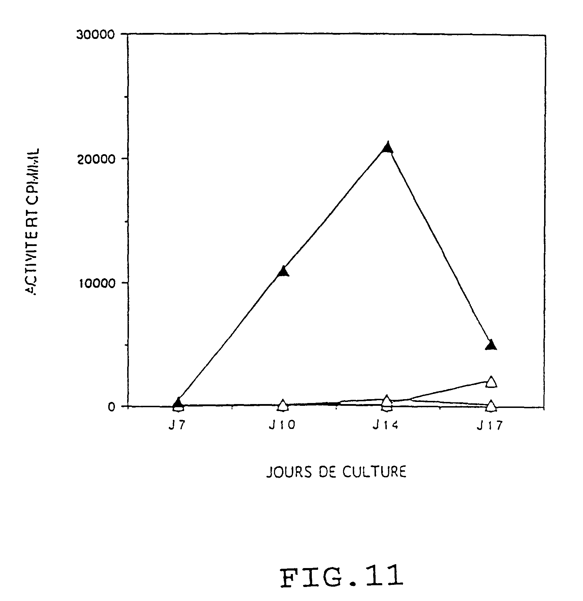

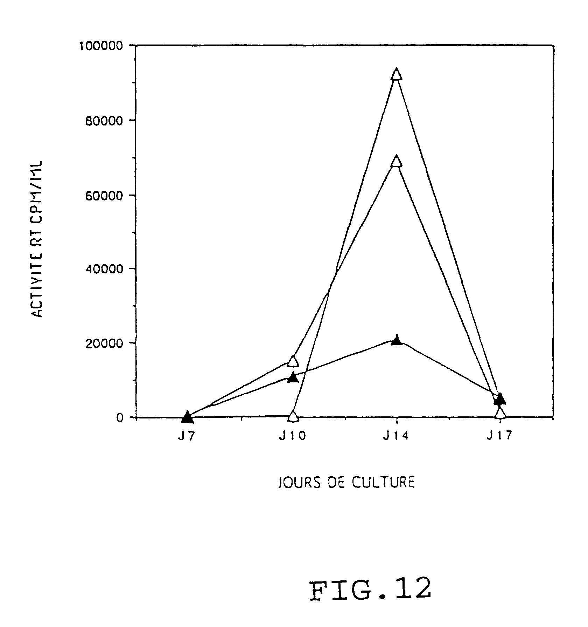

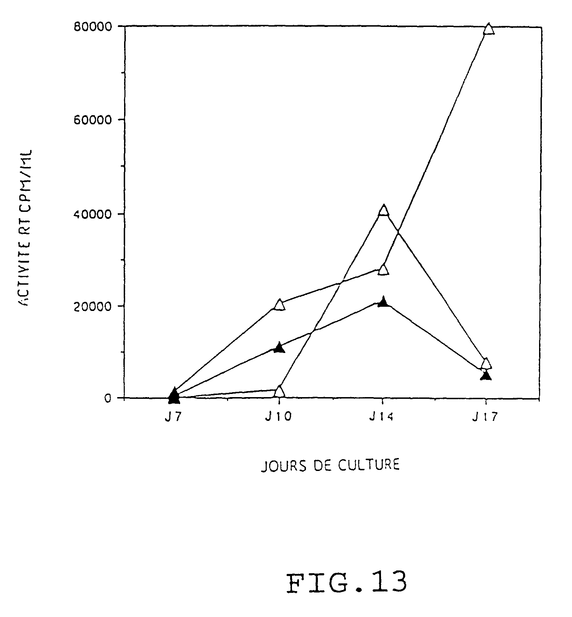

LEGENDE DE LA FIGURE 1A 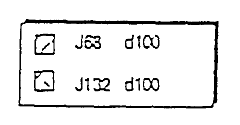
LEGENDE DE LA FIGURE 1B 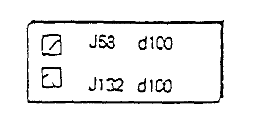
LEGENDE DE LA FIGURE 2
   ELISA avec l'antisérum de lapin pour R7V ou β2m
   lapin 618 (immunisation avec R7V-KLH) dilution sérum 1/100 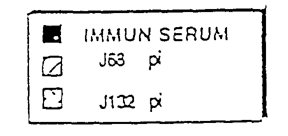
LEGENDE DE LA FIGURE 3A
   ELISA avec de l'antisérum de lapin sur des puits revêtus de peptide 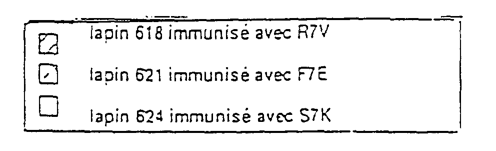
LEGENDE DE LA FIGURE 3B
   ELISA avec des sera de lapins immunisés 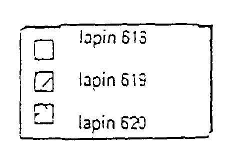
LEGENDE DE LA FIGURE 3C
   ELISA avec des sera de lapins immunisés 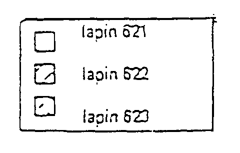
LEGENDE DE LA FIGURE 3D
   ELISA avec des sera de lapins immunisés 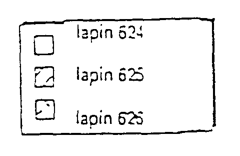
LEGENDE DE LA FIGURE 4
   ELISA avec B1G6, C21.43, B2G2.2 mAb pour R7V 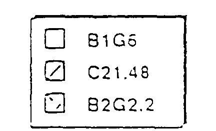
LEGENDE DE LA FIGURE 5
   ELISA POUR R7V-BSA OU β2 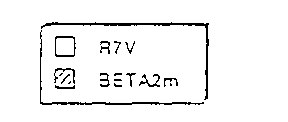
LEGENDE DE LA FIGURE 6
   NEUTRALISATION DE HIV-1 BRU-1 PAR LE SERUM MEC EV (1/50) SUR PBL 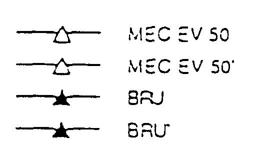
LEGENDE DE LA FIGURE 7
   NEUTRALISATION DE HIV-1 BRU-1 PAR LE SERUM DE BUB JE (1/50) SUR PBL 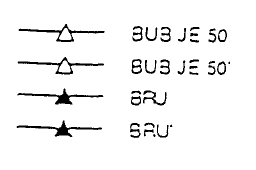
LEGENDE DE LA FIGURE 8
   EFFET DU SERUM SAU CH (1/50) SUR LA PRODUCTION DE HIV-1 BRU-1 SUR PBL 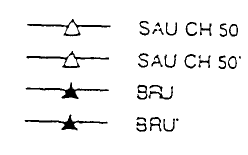
LEGENDE DE LA FIGURE 9
   EFFET D'UN SERUM DE PATIENT HIV- SUR LA PRODUCTION DE HIV-1 SUR PBL 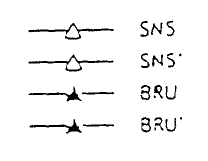
LEGENDE DE LA FIGURE 10
   NEUTRALISATION DE HIV-1 NDK PAR LE SERUM MEC EV (1/50) SUR PBL 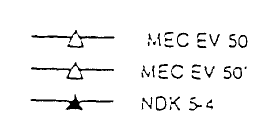
LEGENDE DE LA FIGURE 11
   NEUTRALISATION DE HIV-1 NDK PAR LE SERUM BUB JE (1/50) SUR PBL 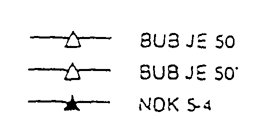
LEGENDE DE LA FIGURE 12
   EFFET DU SERUM SAU CH (1/50) SUR LA PRODUCTION DE HIV-1 NDK SUR PBL 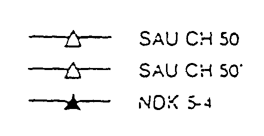
LEGENDE DE LA FIGURE 13
   EFFET D'UN SERUM DE PATIENT HIV- SUR LA PRODUCTION DE HIV-1 NDK SUR PBL

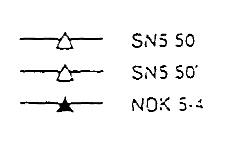

## Revendications

1. Vaccin destiné au traitement et/ou à la prévention de maladies d'origine infectieuse, l'agent infectieux ayant au moins une phase intracellulaire chez l'hôte lors de son cycle de multiplication, **caractérisé en ce qu'**il comporte au moins un épitope cryptique d'un élément cellulaire emporté par un agent infectieux intracellulaire lors de son passage à l'extérieur de la cellule et qui est dévoilé par l'agent infectieux.

2. Vaccin selon la revendication 1, **caractérisé en ce que** l'agent infectieux est constitué par un parasite intracellulaire ou un virus à enveloppe.

3. Vaccin selon la revendication 2, **caractérisé en ce que** le virus est choisi parmi HIV, CMV et HPV.

4. Composition pharmaceutique destinée au traitement ou à la prévention des infections à HIV, **caractérisée en ce qu'**elle comporte à titre de principe actif au moins un peptide correspondant aux séquences ID NO: 1 à 22.

5. Composition selon la revendication 4, **caractérisée en ce que** le peptide est lié à un système porteur.

6. Composition selon la revendication 5, **caractérisée en ce que** le système porteur est constitué d'un ou plusieurs fragments de protéine liés à l'extrémité N ou C terminale du peptide par une liaison peptidique.

7. Composition selon la revendication 4, **caractérisée en ce que** le système porteur est lié au peptide par une liaison non peptidique.

8. Composition selon l'une des revendications 4 à 7, **caractérisée en ce que** le peptide a la séquence R7V.

9. Composition selon l'une des revendications 4 à 8, **caractérisée en ce qu'**elle comporte plusieurs peptides.

10. Composition selon l'une des revendications 4 à 9, **caractérisée en ce que** le système porteur est choisi parmi les albumines, la KLH (Keyhole Limpet Hemocynanin) et la MPA (Multiple Antigenic Peptide).

11. Composition selon l'une des revendications 4 à 10, **caractérisée en ce qu'**elle comporte en outre au moins un adjuvant d'immunité non spécifique.

12. Composition pharmaceutique destinée au traitement et à la prévention des infections à HIV, **caractérisée en ce qu'**elle comporte au moins une séquence d'ADN codant pour un peptide selon l'une des séquences ID NO: 1 à 22.

13. Composition selon la revendication 12, **caractérisée en ce que** la séquence d'ADN est précédée de la séquence assurant son expression dans une cellule hote.

14. Composition selon l'une des revendications 12 et 13, **caractérisée en ce que** la séquence d'ADN est portée par un vecteur d'expression.

15. Composition selon la revendication 14, **caractérisée en ce que** le vecteur d'expression est en réplication autonome.

16. Composition selon la revendication 14, **caractérisée en ce que** le vecteur d'expression est un vecteur d'intégration chromosomique.

17. Composition selon l'une des revendications 14 à 16, **caractérisée en ce que** le vecteur d'expression est un plasmide bactérien.

18. Composition selon l'une des revendications 14 à 17, **caractérisée en ce que** le vecteur d'expression est constitué de tout ou partie d'un virus défectif et/ou non pathogène.

19. Composition selon l'une des revendications 13 à 18, **caractérisée en ce que** le peptide est exprimé dans une cellule hôte.

20. Composition selon la revendication 19, **caractérisée en ce que** ladite cellule est une cellule eucaryote ou végétale.

21. Utilisation d'un anticorps dirigé contre un peptide constitutif d'une composition selon l'une des revendications 4 à 11 pour préparer un médicament destiné au traitement du SIDA.

22. Composition pharmaceutique comportant au moins un anticorps dirigé contre un peptide constitutif d'une composition selon l'une des revendications 4 à 11.

23. Procédé de diagnostic de patients non progresseurs **caractérisé en ce que** l'on détecte par un test immunologique la présence d'un anticorps dirigé contre un peptide constitutif d'une composition selon l'une des revendications 4 à 11.

24. Procédé selon la revendication 23, **caractérisé en ce que** le test immunologique est un test ELISA ou RIA.

## Patentansprüche

1. Vakzine, bestimmt zur Behandlung und/oder Verhütung von Krankheiten infektiösen Ursprungs, wobei das infektiöse Agens bei seinem Multiplikationszyklus mindestens eine intrazelluläre Phase beim Wirt aufweist, **dadurch gekennzeichnet, dass** es mindestens einen kryptischen Epitop eines zellulären Elements umfasst, der von einem intrazellulären infektiösen Agens bei seinem Durchtritt zum Äußeren der Zelle mitgenommen wird und der durch das infektiöse Agens enthüllt wird.

2. Vakzine nach Anspruch 1, **dadurch gekennzeichnet, dass** das infektiöse Agens ein intrazellulärer Parasit oder ein komplexes Virus ist.

3. Vakzine nach Anspruch 2, **dadurch gekennzeichnet, dass** das Virus aus HIV, CMV und HPV ausgewählt ist.

4. Pharmazeutische Zusammensetzung, bestimmt zur Behandlung oder Verhütung von HIV-Infektionen, **dadurch gekennzeichnet, dass** sie als Wirkstoff mindestens ein Peptid umfasst, welches den Sequenzen ID Nr.: 1 bis 22 entspricht.

5. Zusammensetzung nach Anspruch 4, **dadurch gekennzeichnet, dass** das Peptid an ein Trägersystem geknüpft ist.

6. Zusammensetzung nach Anspruch 5, **dadurch gekennzeichnet, dass** das Trägersystem aus einem oder mehreren Proteinfragmenten besteht, die durch eine Peptidbindung an das äußerste N- oder C-terminale Ende des Peptids geknüpft sind.

7. Zusammensetzung nach Anspruch 5, **dadurch gekennzeichnet, dass** das Trägersystem durch eine nicht-peptidische Bindung an das Peptid gebunden ist.

8. Zusammensetzung nach einem der Ansprüche 4 bis 7, **dadurch gekennzeichnet, dass** das Peptid die Sequenz R7V aufweist.

9. Zusammensetzung nach einem der Ansprüche 4 bis 8, **dadurch gekennzeichnet, dass** sie mehrere Peptide umfasst.

10. Zusammensetzung nach einem der Ansprüche 4 bis 9, **dadurch gekennzeichnet, dass** das Trägersystem aus Albuminen, KLH (Keyhole Limpet Hemocyanin) und MPA (Multiple Antigenic Peptide) ausgewählt ist.

11. Zusammensetzung nach einem der Ansprüche 4 bis 10, **dadurch gekennzeichnet, dass** sie darüber hinaus mindestens ein nicht-spezifisches Immunadjuvans umfasst.

12. Pharmazeutische Zusammensetzung, bestimmt zur Behandlung und Verhütung von HIV-Infektionen, **dadurch gekennzeichnet, dass** sie mindestens eine DNS-Sequenz umfasst, welche für ein Peptid gemäß einer der Sequenzen ID Nr.: 1 bis 22 kodiert.

13. Zusammensetzung nach Anspruch 12, **dadurch gekennzeichnet, dass** der DNS-Sequenz die Sequenz vorangeht, welche ihre Expression in einer Wirtszelle sicherstellt.

14. Zusammensetzung nach einem der Ansprüche 12 und 13, **dadurch gekennzeichnet, dass** die DNS-Sequenz durch einen Expressionsvektor getragen wird.

15. Zusammensetzung nach Anspruch 14, **dadurch gekennzeichnet, dass** der Expressionsvektor in autonomer Replikation vorliegt.

16. Zusammensetzung nach Anspruch 14, **dadurch gekennzeichnet, dass** der Expressionsvektor ein Vektor mit Chromosomenintegration ist.

17. Zusammensetzung nach einem der Ansprüche 14 bis 16, **dadurch gekennzeichnet, dass** der Expressionsvektor ein Bakterienplasmid ist.

18. Zusammensetzung nach einem der Ansprüche 14 bis 17, **dadurch gekennzeichnet, dass** der Expressionsvektor aus dem Ganzen oder einem Teil eines defekten und/oder nicht-pathogenen Virus aufgebaut ist.

19. Zusammensetzung nach einem der Ansprüche 13 bis 18, **dadurch gekennzeichnet, dass** das Peptid in einer Wirtszelle exprimiert wird.

20. Zusammensetzung nach Anspruch 19, **dadurch gekennzeichnet, dass** die Zelle eine eukaryotische oder pflanzliche Zelle ist.

21. Verwendung eines Antikörpers, der gegen einen Peptid-Bestandteil einer Zusammensetzung gemäß einem der Ansprüche 4 bis 11 gerichtet ist, um ein Medikament herzustellen, das für die Behandlung von AIDS bestimmt ist.

22. Pharmazeutische Zusammensetzung, umfassend mindestens einen Antikörper, der gegen einen Peptid-Bestandteil einer Zusammensetzung gemäß einem der Ansprüche 4 bis 11 gerichtet ist.

23. Verfahren zur Diagnostik von Patienten, deren Krankheit nicht fortschreitet, **dadurch gekennzeichnet, dass** man durch einen immunologischen Test die Anwesenheit eines Antikörpers nachweist, der gegen einen Peptid-Bestandteil einer Zusammensetzung gemäß einem der Ansprüche 4 bis 11 gerichtet ist.

24. Verfahren nach Anspruch 23, **dadurch gekennzeichnet, dass** der immunologische Test ein ELISA- oder RIA-Test ist.

## Claims

1. Vaccine intended for the treatment and/or prevention of diseases of infectious origin, the infectious agent having at least one intracellular phase in the host during its multiplication cycle, **characterized in that** it comprises at least one cryptic epitope of a cellular component carried away by an intracellular infectious agent during its passage outside the cell and which is exposed by the infectious agent.

2. Vaccine according to Claim 1, **characterized in that** the infectious agent consists of an intracellular parasite or an enveloped virus.

3. Vaccine according to Claim 2, **characterized in that** the virus is chosen from HIV, CMV and HPV.

4. Pharmaceutical composition intended for the treatment or for the prevention of HIV infections, **characterized in that** it comprises, as active ingredient, at least one peptide corresponding to sequences ID NO: 1 to 22.

5. Composition according to Claim 4, **characterized in that** the peptide is bound to a carrier system.

6. Composition according to Claim 5, **characterized in that** the carrier system consists of one or more protein fragments linked to the N- or C-terminal end of the peptide by a peptide bond.

7. Composition according to Claim 4, **characterized in that** the carrier system is linked to the peptide by a nonpeptide bond.

8. Composition according to one of Claims 4 to 7, **characterized in that** the peptide has the R7V sequence.

9. Composition according to one of Claims 4 to 8, **characterized in that** it comprises several peptides.

10. Composition according to one of Claims 4 to 9, **characterized in that** the carrier system is chosen from albumins, KLH (Keyhole Limpet Hemocyanin) and MAP (Multiple Antigenic Peptide).

11. Composition according to one of Claims 4 to 10, **characterized in that** it comprises, in addition, at least one nonspecific immunity adjuvant.

12. Pharmaceutical composition intended for the treatment and prevention of HIV infections, **characterized in that** it comprises at least one DNA sequence encoding a peptide according to one of the sequences ID NO: 1 to 22.

13. Composition according to Claim 12, **characterized in that** the DNA sequence is preceded by the sequence ensuring its expression in a host cell.

14. Composition according to either of Claims 12 and 13, **characterized in that** the DNA sequence is carried by an expression vector.

15. Composition according to Claim 14, **characterized in that** the expression vector is in autonomous replication.

16. Composition according to Claim 14, **characterized in that** the expression vector is a vector for chromosomal integration.

17. Composition according to one of Claims 14 to 16, **characterized in that** the expression vector is a bacterial plasmid.

18. Composition according to one of Claims 14 to 17, **characterized in that** the expression vector consists of all or part of a defective and/or nonpathogenic virus.

19. Composition according to one of Claims 13 to 18, **characterized in that** the peptide is expressed in a host cell.

20. Composition according to Claim 19, **characterized in that** said cell is a eukaryotic or plant cell.

21. Use of an antibody directed against a constituent peptide of a composition according to one of Claims 4 to 11 for preparing a medicament intended for the treatment of AIDS.

22. Pharmaceutical composition comprising at least one antibody directed against a constituent peptide of a composition according to one of Claims 4 to 11.

23. Method for diagnosing nonprogressing patients, **characterized in that** the presence of an antibody directed against a constituent peptide of a composition according to one of Claims 4 to 11 is detected by an immunological test.

24. Method according to Claim 23, **characterized in that** the immunological test is an ELISA or RIA test.
